# EUROPÄISCHE PATENTSCHRIFT

(11) **EP 1 139 989 B1**
(45) Veröffentlichungstag und Bekanntmachung des Hinweises auf die Patenterteilung: **18.10.2006**
(21) Anmeldenummer: 99965462.7
(22) Anmeldetag: 14.12.1999
(51) Int. Cl.: A61K 8/34, A61K 8/41, A61K 8/49, A61Q 5/10

(54) **MITTEL ZUM FÄRBEN VON KERATINHALTIGEN FASERN**
AGENT FOR COLORING KERATINACEOUS FIBERS
PRODUITS DE COLORATION DE FIBRES KERATINIQUES

(30) Priorität: 23.12.1998 DE 19859800
(43) Veröffentlichungstag der Anmeldung: 10.10.2001
(73) Patentinhaber: Henkel Kommanditgesellschaft auf Aktien, 40589 Düsseldorf (DE)
(72) Erfinder: HÖFFKES, Horst, D-40595 Düsseldorf (DE); OBERKOBUSCH, Doris, D-40591 Düsseldorf (DE); ROSE, David, D-40723 Hilden (DE); HITZ, Melanie, D-41542 Dormagen (DE)
(86) Internationale Anmeldenummer: PCT/EP1999/009901
(87) Internationale Veröffentlichungsnummer: WO 2000/038629

(56) Entgegenhaltungen:
- EP-A- 0 542 129
- EP-A- 0 657 158
- WO-A-92/19220
- DE-A- 19 535 340
- DE-A- 19 539 264
- DE-A- 19 610 947
- DE-A- 19 614 303
- DE-U- 9 105 827
- DE-U- 29 702 192

## Beschreibung

Die Erfindung betrifft ein Mittel zum Färben von keratinhaltigen Fasern, insbesondere menschlichen Haaren, das Pyrimidinderivate in Kombination mit speziellen Kupplern enthält, die Verwendung dieser Kombination als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2.5.6-Triamino-4-hydroxypyrimidin.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthatin, 5-Amino-2-methylphenol, m-Aminophenol, Resorcin, Resorcinmonomethylether, m-Phenylendiamin, 2,4-diaminophenoxyethanol, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin und 5-Methylresorcin.

In der deutschen Patentanmeldung DE-A1-41 15148 werden Oxidationsfärbemittel offenbart, die in einem kosmetischen Träger, ein 2,4,5,6-Tetraaminopyrimidin oder ein 6-Hydroxy-2,4,5-Triaminopyridin als Oxidationsbase (Entwickler) sowie eine Kombination aus bestimmten Grünkupplern und Violettkupplern zur Erzeugung brillanter und waschechter Schwarzfärbungen enthalten.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsuntemehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z.B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Aufgabe der vorliegenden Erfindung ist es, ein Färbemittel für Keratinfasern, insbesondere menschliche Haare, bereitzustellen, das durch Luftsauerstoff oxidierbar ist, also nicht unbedingt auf Oxidationsmittel, wie z.B. H₂O₂, angewiesen ist. Das Mittel sollte in einfacher Weise auf die Faser aufgebracht werden können und hinsichtlich der Farbtiefe, der Grauabdeckung und den Echtheitseigenschaften qualitativ den sonst üblichen Oxidationshaarfärbemitteln mindestens gleichwertig sind. Darüber hinaus dürfen die Färbemittel kein oder lediglich ein sehr geringes Sensibilisierungspotential aufweisen. Eine weitere Aufgabe war es, ein Färbesystem zu finden, daß es durch speziell aufeinander abgestimmte Komponenten erlaubt, blaue Nuancen auf der keratinhaltigen Faser zu erzeugen.

Überraschenderweise wurde nun gefunden, daß Pyrimindinderivate in Kombination mit speziellen Kupplern sich auch in Abwesenheit von oxidierenden Agentien, d.h. in Gegenwart von Luftsauerstoff, hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agentien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend
A) mindestens ein Pyrimidinderivat mit der allgemeinen Formel I 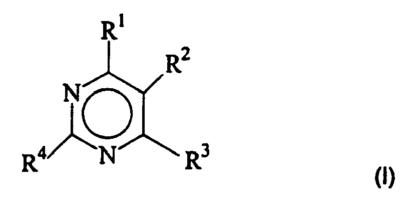 worin R¹, R², R³ und R⁴ gleich oder verschieden sein können und Wasserstoff, OH, NH₂ oder eine Gruppe NR⁵R⁶ sind, in der R⁵ und R⁶gleich oder verschieden sein können und für C₁-C₄-Alkyl, C₁-C₄-Hydroxyalkyl mit primärer und/oder sekundärer Hydroxygruppe stehen,
   wobei zwei der Reste R¹, R², R³ oder R⁴ zusammen einen gegebenenfalls substituierten 5- und 6-gliedrigen Heterocyclus mit einem oder zwei Stickstoff- und/oder Sauerstoffatom(en) im Molekül bilden können,
   mit der Maßgabe, daß mindestens zwei der Reste R¹, R², R³ oder R⁴ eine Gruppe NH₂ und/oder NR⁵R⁶ sind,
B) mindestens eine Verbindung ausgewählt aus der Gruppe bestehend aus
   (a) m-Phenylenderivaten mit den Formeln II und III 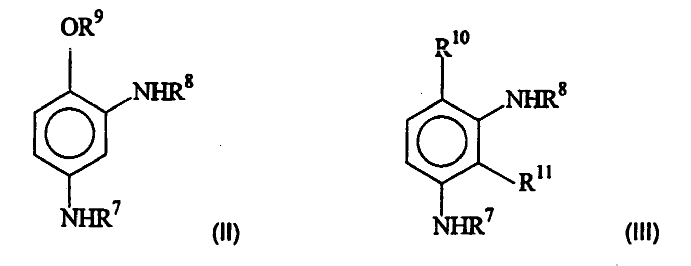 worin R⁷ und R⁸ gleich oder verschieden sein können und für Wasserstoff, C₁-C₄-Alkyl oder C₁-C₄-Hydroxyalkyl stehen,
      R⁹ für C₁-C₄-Hydroxyalkyl oder einen Rest mit der allgemeinen Formel IV steht 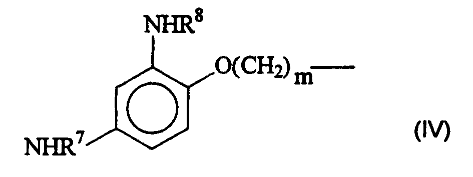 worin R⁷ und R⁸ wie oben definiert sind und m eine ganze Zahl von 1 bis 4 bedeutet,
      R¹⁰ für Wasserstoff oder einen Rest mit der allgemeinen Formel V steht 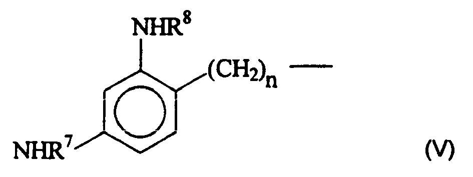 worin R⁷ und R⁸ wie oben definiert sind und n eine ganze Zahl von 1 bis 4 bedeutet,
      R¹¹ für Wasserstoff, C₁-C₄-Alkyl oder C₁-C₄-Hydroxyalkyl steht,
   (b) m-Aminophenolderivaten 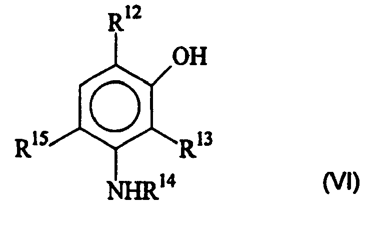 worin R¹² für Wasserstoff oder C₁-C₄-Alkyl steht,
      R¹³ für Wasserstoff, Fluor, Chlor, OCH₃ oder C₁-C₄-Alkyl steht,
      R¹⁴ für Wasserstoff, C₁-C₄-Alkyl, C₁-C₄-Hydroxyalkyl oder OCF₃ steht,
      R¹⁵ für Wasserstoff, Fluor, Chlor oder OCH₃ steht,
      mit den Maßgaben, daß R¹², R¹³, R¹⁴ und R¹⁵ nicht gleichzeitig Wasserstoff sind und daß, wenn R¹² Methyl ist, R¹³, R¹⁴ und R¹⁵ nicht gleichzeitig Wasserstoff sind,
   (c) Pyridinderivaten mit den Formeln VH und VIII 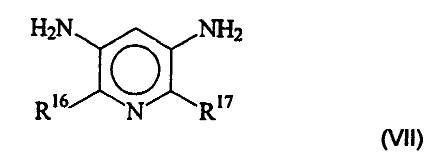 worin R¹⁶ und R¹⁷ gleich oder verschieden sein können und für Fluor, Chlor oder OCH₃ stehen, 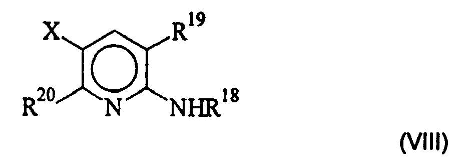 worin R¹⁸ für Wasserstoff, C₁-C₄-Alkyl oder C₁-C₄-Hydroxyalkyl steht,
      R¹⁹ für OH oder NH₂ steht,
      R²⁰ für Wasserstoff, C₁-C₄-Alkoxy oder NH₂ steht,
      X für Wasserstoff oder OCH₃ steht,
      mit den Maßgaben, daß, wenn R¹⁹ NH₂ ist, R¹⁸und R²⁰ nicht gleichzeitig C₁-C₄-Alkyl beziehungsweise Methoxy sind, und wenn R¹⁸ Wasserstoff ist, R¹⁹ und R²⁰ nicht gleichzeitig OH beziehungsweise Wasserstoff sind,
   (d) Resorcinderivaten mit der Formel IX 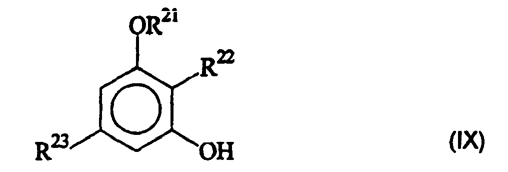 worin R²¹, R²² und R²³ gleich oder verschieden sein können und für Wasserstoff, C₁₋C₄-Alkyl oder C₁-C₄-Hydroxyalkyl stehen,
      mit den Maßgaben, daß R²¹, R²² und R²³ nicht gleichzeitig Wasserstoff sind, wenn R²¹ und R²³ Wasserstoff sind, R²² nicht Methyl ist, und wenn R²¹ für Methyl steht, R²² und R²³ nicht gleichzeitig Wasserstoff sind,
   (e) Methylendioxybenzolderivaten
      ausgewählt aus 3,4-Methylendioxyphenol, 3,4-Methylendioxyanilin und N-(2-Hydroxyethyl)-3,4-methylendioxyanilin (f) 3,4-Diaminobenzoesäure, und
C) aktivierte Carbonylverbindungen sowie weitere als Entwickler oder Kuppler bekannt Substanzen.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl- oder Acetylcellulose und synthetischer Fasern, wie z.B. Polyamid-, Polyacrytnitril,-, Polyurethan- und Polyesterfasern verwendet werden.

Die erfindungsgemäß eingesetzten Pyrimidinderivate mit der Formel I sind vorzugsweise ausgewählt aus der Gruppe bestehend aus 4-Hydroxy-2,5,6-triaminopyrimidin, 2-Hydroxy-2,5,6-triaminopyrimidin, 2,4,5,6-Tetraaminopyrimidin, 5,6-Diamino-2,4-dihydroxypyrimidin, 2,4-Diamino-5,6-dihydroxypyrimidin, 4-Dimethylamino-2,5,6-tetraminopyrimidin. Besonders bevorzugt werden 2,4,5,6-Tetraaminopyrimidin, 4-Dimethylamino-2,5,6-tetraminopyrimidin, 4-Hydroxy-2,5,6-triaminopyrimidin und 5,6-Diamino-2,4-dihydroxypyrimidin eingesetzt.

Diese Substanzen sind litetaturbekannt oder im Handel erhältlich.

Die voranstehend genannten Pyrimidinderivate mit der Formel I werden vorzugsweise in den erfindungsgemäßen Mitteln in einer Menge von 0,03 bis 65 mmol, insbesondere von 1 bis 40 mmol, bezogen auf 100 g des gesamten Färbemittels, verwendet.

Unter den Verbindungen der Formel VIII sind diejenigen bevorzugt, bei denen X für Wasserstoff steht.

Kuppler der Komponente B sind vorzugsweise ausgewählt aus der Gruppe 1,3-Bis(2,4-diaminophenoxypropan), 1,3-Bis-(2,4-diaminophenylpropan), 2,4-Diaminophenoxyethanol, 2,6-Bis-(2'-hydroxyethylamino)-toluol, 3-Amino-2-chlor-6-methyl-phenol, 5-Amino-4-chlor-2-methylphenol, 2,4-Dichlor-3-amino-phenol, 3,5-Diamino-2,6-dimethoxypyridin, 5-Methylresorcin, 2,5-Dimethylresorcin, 3,4-Methylendioxyphenol, 3,4-Methylendioxyanilin, N-(2-Hydroxyethyl)-3,4-methylendioxyanilin und beliebigen Gemischen der voranstehenden.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene Pyrimidinderivate der Formel I gemeinsam zum Einsatz kommen; ebenso können auch mehrere verschiedene Verbindungen der Komponente B gemeinsam verwendet werden. Unter diese Ausführungsform fällt auch die Verwendung von solchen Substanzen, die Reaktionsprodukte von Pyrimidinderivaten der Formel I mit den genannten Verbindungen der Komponente B darstellen.

Die Farbnuancen können noch weiter variiert und vertieft werden, wenn dem erfindungsgemäßen Mittel eine oder mehrere Verbindungen ausgewählt aus 5,6-Dihydroxyindol sowie dessen N-substituierten C₁-C₄-Alkyl- und C₁-C₄-Hydroxyalkylderivaten, 5,6-Dihydroxyindolin sowie dessen N-substituierten C₁-C₄-Alkyl- und C₁-C₄₋Hydroxyalkylderivaten und den als Entwickler bekannten Verbindungen, ausgewählt aus der Gruppe bestehend aus p-Phenylendiamin, p-Toluylendiamin, p-Aminophenol, 4,4'-Diamino-diphenylamin, 1,10-Bis-(2,5-diaminophenyl)-1,4,7,10-tetraoxydecan, 2,(2'-Hydroxyethyl)-p-phenylendiamin, 2,6-Dichlor-4-aminophenol, N,N-Bis-(2'-hydroxyethyl)-p-phenylendiamin, 4-Amino-3-methylphenol, 2-Aminomethyl-4-aminophenol, 5-Amino-salicylsäure, Bis-(2-Hydroxy-5-aminophenyl)-methan und 2-(2,5-Diaminophenoxy)ethanol, zugesetzt werden.

Eine weitere bevorzugte Entwicklerkomponente ist 4-Amino-2-((diethytamino)-methyl)-phenol.

Besonders bevorzugte Entwicklerkomponenten sind beispielsweise p-Phenylendiamin, p-Toluylendiamin, 1,10-Bis-(2,5-diaminophenyl)-1,4,7,10-tetraoxydecan, 2,(2'-Hydroxyethyl)-p-phenylendiamin, 2,6-Dichlor-4-aminophenol, N,N-Bis-(2'-hydroxyethyl)-p-phenylendiamin, 3-Methyl-4-aminophenol, 2-Aminomethyl-4-aminophenol, 4-Amino-2-((diethylamino)-methyl)-phenol und Bis-(2-Hydroxy-5-aminophenyl)-methan.

In einer weiteren bevorzugten Ausführungsform werden der erfindungsgemäßen Kombination aus den Komponenten A und B zur weiteren Modifizierung der Farbnuancen aktivierte Carbonylverbindungen sowie weitere als Entwickler oder Kuppler bekannte Substanzen zugesetzt.

Beispiele für aktivierte Carbonylverbindungen sind Isatin, 5-Chlorisatin, 5-Bromisatin, 6-Bromisatin, 5-Nitroisatin, N-Hydroxymethylisatin, N-Allylisatin, 5-Isatinsulfonsäure-Na-salz, Glutaconaldehyd-tetrabutylammonium-salz, Tribasenaldehyd, Malonaldehyd-bis-dimethylacetal, 4-Hydroxy-3-methoxycinnan-aldehyd, 1-Piperidino-methylisatin, 1-Diethylamino-methylisatin, Glutaconaldehyd-Na-salz, 5-N-methylanilinopentadienal, 2-Chlor-3-hydroxymethylen-1-cyclohexen-1-aldehyd, N-(5-Anilino-2,4-pentandien-1-yliden)-anilinium-chlorid, trans-β-(2-Furyl)acrolein, 2-Nitro-1,3-indandion, Dehydroascorbinsäure, 2-Acetyl-1,3-cyclohexandion, 7-Dimethylamino-2,4,6-heptatrienyliden-dimethylamonium-perchlorat und 4-Formy-1-methylpyridinium-benzol-sulfonat.

Beispiele für Kuppler, die zusätzlich enthalten sein können, sind 3-Amino-2-methylamino-6-methoxypridin, 2-Amino-4-(2'-Hydroxyethylamino)-anisol, α-Naphthol, Resorcin, Resorcinmonomethylether, 4-Chlorresorcin, 2-Methylresorcin, m-Aminophenol, 3-N,N,-Dimethyl-aminophenol, 5-Amino-2-methoxyphenol, 5-Amino-2-methylphenol, 3-Amino-2,4-dimethylphenol, 3-(N-Cyclopentyl)-aminophenol, 1,5-, 1,7-, 2,7-Dihydroxynaphthaline, o-Aminophenol, 6-Hydroxybenzomorpholin, 1-Phenyl-3-methyl-pyrazol-5-on, 2-Amino-6-methylphenol, 2,6-Dihydroxy-3,4-dimethyl-pyridin, 4-Hydroxyindol, 6-Hydroxyindol, 7-Hydroxyindol, 4-Aminoindol und 2,4-Diamino-5-methylphenetol.

Das erfindungsgemäße Färbemittel stellt ein luftoxidables System dar. Auf die Anwesenheit von zusätzlichen Oxidationsmitteln, z.B. H₂O₂, kann dabei verzichtet werden. Es kann jedoch u.U. wünschenswert sein, den erfindungsgemäßen Mitteln zur Erzielung der Nuancen, die heller als die zu färbende keratinhaltige Faser sind, Wasserstoffperoxid oder andere Oxidationsmittel, wie Peroxidisulfat oder Percarbonat, zuzusetzen. Weiterhin ist es unter Umständen möglich, in Abhängigkeit vom Oxidationsmittel, d.h. ob Luftsauerstoff oder Wasserstoffperoxid eingesetzt wird, unterschiedliche Farbnuancen einzustellen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

Weiterhin ist es möglich, die Oxidation mit Hilfe von Enzymen durchzuführen. Dabei können die Enzyme sowohl zur Erzeugung von oxidierenden Per-Verbindungen eingesetzt werden, als auch zu Verstärkung der Wirkung einer geringen Menge vorhandener Oxidationsmittel. Beispiele für enzymatische Verfahren sind die Verwendung von Laccasen sowie die Verstärkung der Wirkung geringer Mengen (z. B. 1 % und weniger, bezogen auf das gesamte Mittel) Wasserstoffperoxid durch Peroxidasen.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z.B. aus der Gruppe der Nitrophenylendiamine, Nitroaminophenole, Anthrachinone oder Indophenole, wie z.B. die unter den internationalen Bezeichnungen bzw. Handelsnamen HC Yellow 2, HC Yellow 4, HC Yellow 6. Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Ammo-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auchzwitterionische, ampho-Iytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂₋Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freieAminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 949 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise VinylpyrrolidonNinylacrylat-Copolymere, Polyvinylpyrrolidon und VinylpyrrolidonNinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobomytacrytat-Copolymere, Methylvinylether/Maleinsäureanhydrid-Copolymere und Acrytsäure/Ethylacrylat/N-tert.-Butylacrylamid-Terpolymere.
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittier wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z.B. Formiate, Carbonate, Halogenide, Sulfate, Butyrate, Valeriate, Capronate, Acetate, Lactate, Glykolate,Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Ein weiterer Gegenstand der vorliegenden Erfindung betrifft die Verwendung einer Kombination aus
A) mindestens einem Pyrimidinderivat mit der allgemeinen Formel I 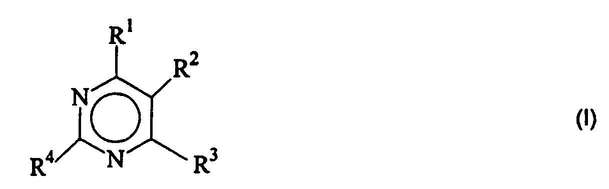 worin R¹, R², R³ und R⁴ gleich oder verschieden sein können und Wasserstoff, OH, NH₂ oder eine Gruppe NR⁵R⁶ sind, in der R⁵ und R⁶ gleich oder verschieden sein können und für C₁-C₄-Alkyl, C₁-C₄-Hydroxyalkyl mit primärer und/oder sekundärer Hydroxygruppe stehen,
   wobei zwei der Reste R¹, R², R³ oder R⁴ zusammen einen gegebenenfalls substituierten 5- und 6-gliedrigen Heterocyclus mit einem oder zwei Stickstoff- und/oder Sauerstoffatom(en) im Molekül bilden können,
   mit der Maßgabe, daß mindestens zwei der Reste R¹, R², R³ oder R⁴ eine Gruppe NH₂ und/oder NR⁵R⁶ sind,
B) mindestens einer Verbindung ausgewählt aus der Gruppe bestehend aus den oben dargestellten (a) m-Phenylendiaminderivaten mit den allgemeinen Formeln II oder III, (b) m-Aminophenolderivaten der allgemeinen Formel VI, (c) Pyridinderivaten mit den Formeln VII oder VIII, (d) Resorcinderivaten der Formel IX, (e) Methyldioxybenzolderivaten und
C) aktivierte Carbonylverbindungen sowie weitere als Entwickler und Kuppler bekannte Substanzen. oder (f) 3,4-Diaminobenzoesäure, zum Färben von keratinhaltigen Fasern.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend
A) mindestens ein Pyrimidinderivat mit der allgemeinen Formel I 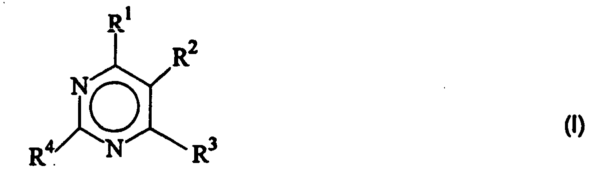 worin R¹, R², R³ und R⁴ gleich oder verschieden sein können und Wasserstoff, OH, NH₂ oder eine Gruppe NR⁵R⁶ sind, in der R⁵ und R⁶ gleich oder verschieden sein können und für C₁-C₄-Alkyl, C₁-C₄-Hydroxyalkyl mit primärer und/oder sekundärer Hydroxygruppe stehen,
   wobei zwei der Reste R¹, R², R³ oder R⁴ zusammen einen gegebenenfalls substituierten 5- und 6-gliedrigen Heterocyclus mit einem oder zwei Stickstoff- und/oder Sauerstoffatom(en) im Molekül bilden können,
   mit der Maßgabe, daß mindestens zwei der Reste R¹, R², R³ oder R⁴ eine Gruppe NH₂ und/oder NR⁵R⁶ sind,
B) mindestens eine Verbindung ausgewählt aus der Gruppe bestehend aus den (a) m-Phenylendiaminderivaten mit den allgemeinen Formeln II oder III, (b) m-Aminophenolderivaten der allgemeinen Formel VI, (c) Pyridinderivaten mit den Formeln VII oder VIII, (d) Resorcinderivaten der Formel IX, (e) den oben genannten Methyldioxybenzolderivaten ,
   und
C) aktivierte Carbonylverbindungen sowie weitere als Entwickler und Kuppler bekannte Substanzen,
   oder (f) 3,4-Diaminobenzoesäure,
   sowie übliche kosmetische Inhaltsstoffe auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die Pyrimidinderivate der Formel I und die Verbindungen der Komponente B können entweder gleichzeitig auf das Haar aufgebracht werden oder aber auch nacheinander, wobei es unerheblich ist, welche der beiden Komponenten zuerst aufgetragen wird. Falls es zur Erlangung eines bestimmten Farbtons erforderlich ist, wird in dieser Stufe auch ggf. eingesetztes Oxidationsmittel gemeinsam mit den anderen Komponenten oder danach aufgebracht. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

### Beispiele

Es wurden erfindungsgemäße Haarfärbemittel in Form einer Haarfärbe-Cremeemulsion der in Tabelle 1 dargestellten Zusammensetzung.

**Tabelle 1**

| | 1* | 2* | 3* | 4 | 5 | 6* | 7* |
|---|---|---|---|---|---|---|---|
| Komponente | Gew.-% | | | | | | |
| C₁₂-C₁₄-Fettalkohol +2 EO-sulfat, Na-Salz, 28-%ige Lösung | 20,0 | 20,0 | 20,0 | 20,0 | 20,0 | 20,0 | 20,0 |
| Kokosamidopropylbetain, 30-%ig | 12,5 | 12,5 | 12,5 | 12,5 | 12,5 | 12,5 | 12,5 |
| C₁₀-C₁₈-Fettalkohol | 2,0 | 2,0 | 2,0 | 2,0 | 2,0 | 2,0 | 2,0 |
| Talgfettalkohol, hydriert | 8,5 | 8,5 | 8,5 | 8,5 | 8,5 | 8,5 | 8,5 |
| C₁₆-C₁₈-Fettalkohol + 20 EO | 0,75 | 0,75 | 0,75 | 0,75 | 0,75 | 0,75 | 0,75 |
| Ammoniumsulfat | 0,1 | 0,1 | 0,1 | 0,1 | 0,1 | 0,1 | 0,1 |
| Natriumsulfat | 0,1 | 0,1 | 0,1 | 0,1 | 0,1 | 0,1 | 0,1 |
| 4-Hydroxy-2,5,6-triaminopyrimidin-sulfat | 2,4 | 2,4 | 2,4 | 2,4 | 2,4 | 2,4 | 2,4 |
| 2,4-Diamino-phenoxyethanol-dihydrochlorid | 2,4 | - | - | - | - | - | - |
| 3,5-Diamino-2,6-dimethoxypyridin-dihydrochlorid | - | 2,4 | - | - | - | - | - |
| 1,3-Bis(2,4-diaminophenoxy)-propan-tetrshydrochiond | - | - | 4,7 | 2,12 | 1,18 | - | - |
| N-Allylisatin | - | - | - | 0,19 | 0,94 | - | - |
| 1,10-Bis(2,5-diaminophenyl)-1,4,7-10-tetraoxydecan-tetrahydrochlorid | - | - | - | 1,02 | 5,1 | - | - |
| 3-Amino-2-chlor-6-methylphenol | - | - | - | - | - | 1,58 | - |
| 3.4-Methylendioxyphenol | - | - | - | - | - | - | 1,38 |
| Wasser | ad 100 | | | | | | |

| | | | | | | | |
|---|---|---|---|---|---|---|---|
| * Beispiel wird nicht von den Patentansprüchen umfaßt. | | | | | | | |

Die einzelnen Bestandteile wurden bei 70°C miteinander vermischt und nach dem Abkühlen mit NaOH auf einen pH-Wert von 9,5 eingestellt.

Mit den in Tabelle 1 dargestellen Zusammensetzungen wurden Färbungen unter Verwendung von H₂O₂ ais Oxidationsmittel und ohne Oxidationsmittel, d.h. durch Luftoxidation durchgeführt.

Für die oxidative Entwicklung der Färbung unter Einsatz von H₂O₂ wurden die in Tabelle 1 dargestellten Zusammensetzungen im Verhältnis 1 : 1 mit 12%iger Wasserstoffperoxidlösung vermischt. Im Falle der Luftoxidation wurden die in Tabelle 1 dargestellten Zusammensetzungen vor der Anwendung im Verhältnis 1 : 1 mit Wasser gemischt.

Die Applikationsmischung wurde auf ca. 15 cm lange Strähnen standardisierten, zu 90 % ergrauten, aber nicht besonders vorbehandelten Menschenhaares aufgetragen und dort 30 Minuten bei 27°C belassen. Nach Beendigung des Färbeprozesses wurde das Haar gespült, mit einem üblichen Haarwaschmittel ausgewaschen und anschließend getrocknet. Die Färbeergebnisse sind in Tabelle 2 dargestellt.

**Tabelle 2**

| | Farbnuance | |
|---|---|---|
| Rezeptur-Nr. | H₂O₂-Oxidation | Luftoxidation |
| 1 | tiefes Blauviolett | tiefes Blauviolett |
| 2 | rötliches Mittelbraun | rötliches Mittelbraun |
| 3 | mittel-dunkelbraun | mittel-dunkelbraun |
| 4 | - | Dunkelbraun |
| 5 | - | Schwarz |
| 6 | Graumagenta | kräftiges Bordeaux |
| 7 | Blaßorange | Sandfarben |

Die Färbeergebnisse zeigen, daß die erfindungsgemäßen Mittel sowohl mit als auch ohne Zusatz von Oxidationsmitteln hervorragende Färbeergebnisse liefern. Ähnliche Färbungen werden erhalten, wenn in Beispiel 7 als Oxidationsmittel das EnzymsystemGlucose-Oxidase / Glucose / Peroxidase beziehungsweise das Enzymsystem Uricase / Harnsäure /Peroxidase bei pH = 8,5 eingesetzt wird.

## Patentansprüche

1. Mittel zum Färben von keratinhaltigen Fasern, enthaltend
A) mindestens ein Pyrimidinderivat mit der allgemeinen Formel I 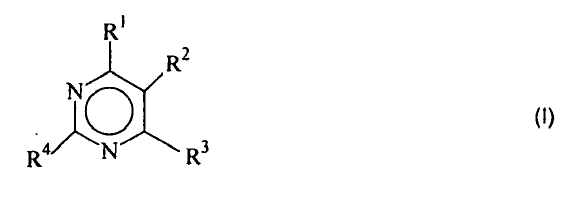 worin R¹, R², R³ und R⁴ gleich oder verschieden sein können und Wasserstoff, OH, NH₂ oder eine Gruppe NR⁵R⁶ sind, in der R⁵ und R⁶ gleich oder verschieden sein können und für C₁-C₄-Alkyl, C₁-C₄-Hydroxyalkyl mit primärer und/oder sekundärer Hydroxygruppe stehen,
wobei zwei der Reste R¹, R², R³ oder R⁴ zusammen einen gegebenenfalls substituierten 5- und 6-gliedrigen Heterocyclus mit einem oder zwei Stickstoff- und/oder Sauerstoffatom(en) im Molekül bilden können, mit der Maßgabe, dass mindestens zwei der Reste R¹, R², R³ oder R⁴ eine Gruppe NH₂ und/oder NR⁵R⁶ sind,
B) mindestens eine Verbindung ausgewählt aus der Gruppe bestehend aus
(a) m-Phenylenderivaten mit den Formeln II und III 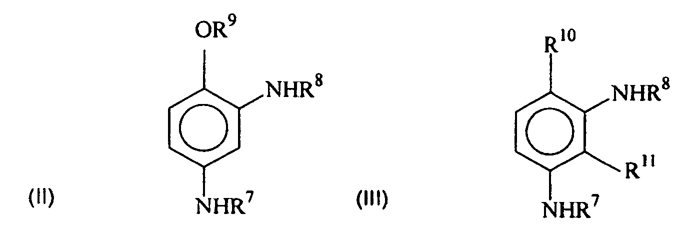 worin R⁷ und R⁸ gleich oder verschieden sein können und für Wasserstoff, C,-C₄-Alkyl oder C₁-C₄-Hydroxyalkyl stehen,
R⁹ für C₁-C₄-Hydroxyalkyl oder einen Rest mit der allgemeinen Formel IV steht 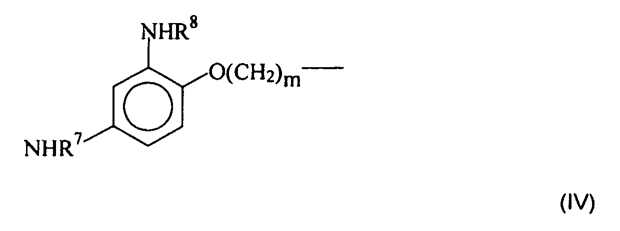 worin R⁷ und R⁸ wie oben definiert sind und m eine ganze Zahl von 1 bis 4 bedeutet,
R¹⁰ für Wasserstoff oder einen Rest mit der allgemeinen Formel V steht 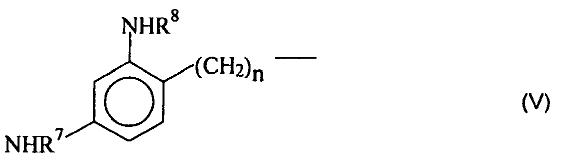 worin R⁷ und R⁸ wie oben definiert sind und n eine ganze Zahl von 1 bis 4 bedeutet,
R¹¹ für Wasserstoff, C₁-C₄-Alkyl oder C₁-C₄-Hydroxyalkyl steht,
(b) m-Aminophenolderivaten 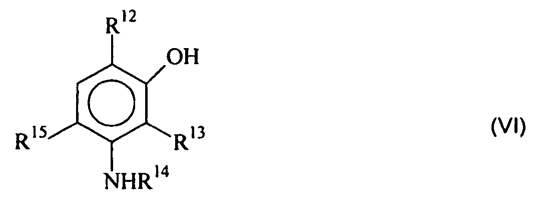 worin R¹² für Wasserstoff oder C₁-C₄-Alkyl steht,
R¹³ für Wasserstoff, Fluor, Chlor, OCH₃ oder C₁-C₄-Alkyl steht,
R¹⁴ für Wasserstoff, C₁-C₄-Alkyl, C₁-C₄-Hydroxyalkyl oder OCF₃ steht,
R¹⁵ für Wasserstoff, Fluor, Chlor oder OCH₃ steht,
mit den Maßgaben, dass R¹², R¹³, R¹⁴ und R¹⁵ nicht gleichzeitig Wasserstoff sind und dass, wenn R¹² Methyl ist, R¹³, R¹⁴ und R¹⁵ nicht gleichzeitig Wasserstoff sind,
(c) Pyridinderivaten mit den Formeln VII und VIII 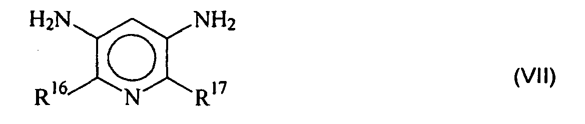 worin R¹⁶ und R¹⁷ gleich oder verschieden sein können und für Fluor, Chlor oder -OCH₃ stehen, 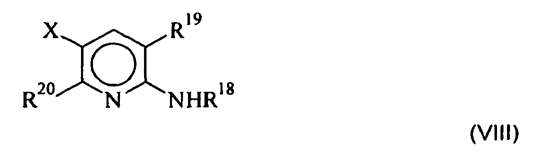 worin R¹⁸ für Wasserstoff, C₁-C₄-Alkyl oder C₁-C₄-Hydroxyalkyl steht,
R¹⁹ für OH oder NH₂ steht,
R²⁰ für Wasserstoff, C₁-C₄-Alkoxy oder NH₂ steht,
X für Wasserstoff oder OCH₃ steht,
mit den Maßgaben, dass, wenn R¹⁹ NH₂ ist, R¹⁶ und R²⁰ nicht gleichzeitig C₁-C₄₋Alkyl beziehungsweise Methoxy sind, und wenn R¹⁸ Wasserstoff ist, R¹⁹ und R²⁰ nicht gleichzeitig OH beziehungsweise Wasserstoff sind,
(d) Resorcinderivaten mit der Formel IX 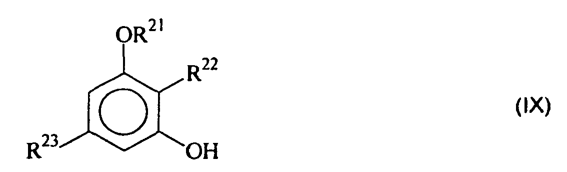 worin R²¹, R²² und R²³ gleich oder verschieden sein können und für Wasserstoff, C₁-C₄-Alkyl oder C₁-C₄-Hydroxyalkyl stehen, mit den Maßgaben, dass R²¹, R²² und R²³ nicht gleichzeitig Wasserstoff sind, wenn R²¹ und R²³ Wasserstoff sind, R²² nicht Methyl ist, und wenn R²¹ für Methyl steht, R²² und R²³ nicht gleichzeitig Wasserstoff sind,
(e) Methylendioxybenzolderivaten, ausgewählt aus 3,4-Methylendioxyphenol, 3,4-Methylendioxyanilin und N-(2-Hydroxyethyl)-3,4-methylendioxyanilin,
(f) 3,4-Diaminobenzoesäure und
C) aktivierte Carbonylverbindungen sowie weitere als Entwickler oder Kuppler bekannte Substanzen.

2. Mittel nach Anspruch 1, **dadurch gekennzeichnet, dass** als Pyrimidinderivate mit der Formel I 4-Hydroxy-2,5,6-triaminopyrimidin, 2-Hydroxy-2,5,6-triaminopyrimidin, 2,4,5,6-Tetraaminopyrimidin, 5,6-Diamino-2,4-dihydroxypyrimidin, 2,4-Diamino-5,6-dihydroxypyrimidin, 4-Dimethylamino-2,5,6-triaminopyrimidin und deren beliebigen Gemische enthalten sind.

3. Mittel nach Anspruch 1 oder 2, **dadurch gekennzeichnet, dass** die Pyrimidinderivate mit der Formel I in einer Menge von 0,03 bis 65 mmol, insbesondere von 1 bis 40 mmol, bezogen auf 100 g des gesamten Färbemittels, enthalten sind.

4. Mittel nach einem der Ansprüche 1 bis 3, **dadurch gekennzeichnet, dass** die Verbindungen der Komponente B ausgewählt sind aus der Gruppe 1,3-Bis(2,4-diaminophenoxypropan), 1,3-Bis-(2,4-diaminophenylpropan), 2,4-Diaminophenoxyethanol, 2,6-Bis-(2'-hydroxyethylamino)-toluol, 3-Amino-2-chlor-6-methyl-phenol, 5-Amino-4-chlor-2-methylphenol, 2,4-Dichlor-3-amino-phenol, 3,5-Diamino-2,6-dimethoxypyridin, 5-Methylresorcin, 2,5-Dimethylresorcin, 3,4-Methylendioxyphenol, 3,4-Methylendioxyanilin, N-(2-Hydroxyethyl)-3,4-methylendioxyanilin und deren beliebigen Gemischen.

5. Mittel nach einem der Ansprüche 1 bis 4, **dadurch gekennzeichnet, dass** die Verbindungen der Komponente B in einer Menge von jeweils 0,03 bis 65, insbesondere 1 bis 40 mmol, jeweils bezogen auf 100 g des gesamten Färbemittels, enthalten sind.

6. Mittel nach einem der Ansprüche 1 bis 5, **dadurch gekennzeichnet, dass** die aktivierte Carbonylverbindung ausgewählt ist aus der Gruppe aus Isatin, 5-Chlorisatin, 5-Bromisatin, 6-Bromisatin, 5-Nitroisatin, N-Hydroxymethylisatin, N-Allylisatin, 5-Isatinsulfonsäure-Na-salz, Glutaconaldehyd-tetrabutylammoniurn-salz, Tribasenaldehyd, Malonaldehyd-bis-dimethylacetal, 4-Hydroxy-3-methoxycinnanaldehyd, 1-Piperidino-methylisatin, 1-Dlethylamino-methylisatin, Glutaconaldehyd-Na-salz, 5-N-methylanilinopentadienal, 2-Chior-3-hydroxymethylen-1-cyclohexen-1-aldehyd, N-(5-Anilino-2,4-pentandien-1-yliden)anilinium-chlorid, trans-β-(2-Furyl)-acrolein, 2-Nitro-1,3-indandion, Dehydroascorbinsäure, 2-Acetyl-1,3-cyclohexandion, 7-Dimethylamino-2,4,6-heptatrienyliden-dimethylamonium-perchlorat und 4-Formyl-1-methylpyridinium-benzol-sulfonat enthalten ist.

7. Mittel nach einem der Ansprüche 1 bis 6, **dadurch gekennzeichnet, dass** weiterhin eine oder mehrere Verbindungen ausgewählt aus 5,6-Dihydroxyindol sowie dessen N-substituierten C₁-C₄-Alkyl- und C₁-C₄-Hydroxyalkylderivate, 5,6-Dihydroxyindolin sowie dessen N-substituierten C₁-C₄-Alkyl- und C₁-C₄-Hydroxyalkylderivaten und den als Entwickler bekannten Verbindungen, ausgewählt aus der Gruppe bestehend aus p-Phenylendiamin, p-Toluylendiamin, p-Aminophenol, 4,4'-Diamino-diphenylamin, 1,10-Bis-(2,5-diaminophenyl)-1,4,7,10-tetraoxydecan, 2,(2'-Hydroxyethyl)-p-phenylendiamin, 2,6-Dichlor-4-aminophenol, N,N-Bis-(2'-hydroxyethyl)-p-phenylendiamin, 3-Methyl-4-aminophenol, 2-Aminomethyl-4-aminophenol, 5-Amino-salicylsäure, Bis-(2-Hydroxy-5-aminophenyl)-methan, 2-(2,5-Diaminophenoxy)-ethanol, zugesetzt werden.

8. Mittel nach einem der Ansprüche 1 bis 7, **dadurch gekennzeichnet, dass** es anionische, zwitterionische oder nichtionische Tenside enthält.

9. Mittel nach einem der Ansprüche 1 bis 8, **dadurch gekennzeichnet, dass** es ein luftoxidables Färbesystem ist.

10. Mittel nach einem der Ansprüche 1 bis 8, **dadurch gekennzeichnet, dass** Oxidationsmittel ausgewählt aus der Gruppe H₂O₂, Peroxidisulfat und Percarbonat enthält.

11. Verwendung einer Kombination aus
A) mindestens einem Pyrimidinderivat mit der allgemeinen Formel I 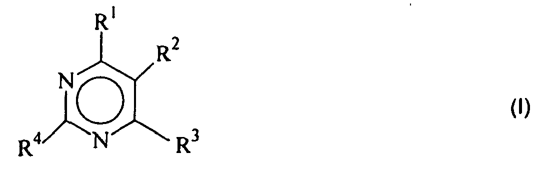 worin R¹, R², R³ und R⁴ gleich oder verschieden sein können und Wasserstoff, OH, NH₂ oder eine Gruppe NR⁵R⁶ sind, in der R⁵ und R⁶ gleich oder verschieden sein können und für C₁-C₄-Alkyl, C₁-C₄-Hydroxyalkyl mit primärer und/oder sekundärer Hydroxygruppe stehen,
wobei zwei der Reste R¹, R², R³ oder R⁴ zusammen einen gegebenenfalls substituierten 5- und 6-gliedrigen Heterocyclus mit einem oder zwei Stickstoff- und/oder Sauerstoffatom(en) im Molekül bilden können,
mit der Maßgabe, dass mindestens zwei der Reste R¹, R², R³ oder R⁴ eine Gruppe NH₂ und/oder NR⁵R⁶ sind,
B) mindestens einer Verbindung ausgewählt aus der Gruppe bestehend aus
(a) m-Phehyienderivaten mit den Formeln II und III 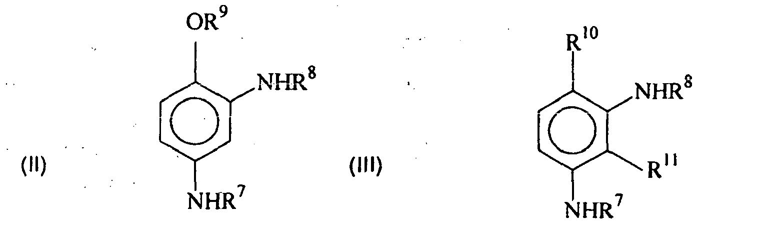 worin R⁷ und R⁸ gleich oder verschieden sein können und für Wasserstoff, C₁₋C₄-Alkyl oder C₁-C₄-Hydroxyalkyl stehen,
R⁹ für C₁-C₄-Hydroxyalkyl oder einen Rest mit der allgemeinen Formel IV steht 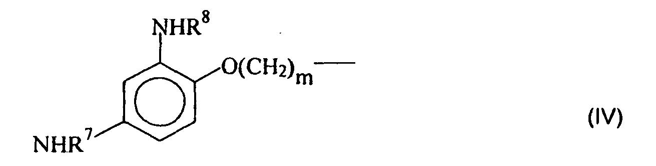 worin R⁷ und R⁸ wie oben definiert sind und m eine ganze Zahl von 1 bis 4 bedeutet,
R¹⁰ für Wasserstoff oder einen Rest mit der allgemeinen Formel V steht 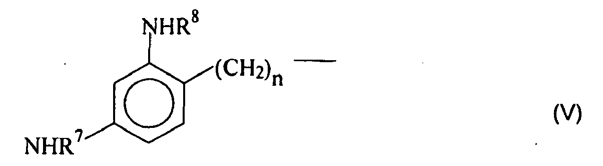 worin R⁷ und R⁸ wie oben definiert sind und n eine ganze Zahl von 1 bis 4 bedeutet,
R¹¹ für Wasserstoff, C₁-C₄-Alkyl oder C₁-C₄-Hydroxyalkyl steht,
(b) m-Aminophenolderivaten 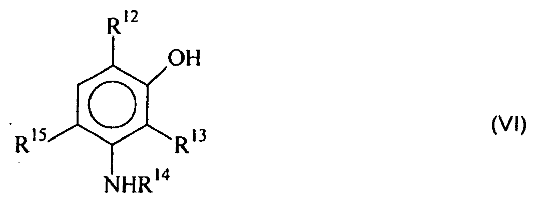 worin R¹² für Wasserstoff oder C₁-C₄-Alkyl steht,
R¹³ für Wasserstoff, Fluor, Chlor, OCH₃ oder C₁-C₄-Alkyl steht,
R¹⁴ für Wasserstoff, C₁-C₄-Alkyl, C₁-C₄-Hydroxyalkyl oder OCF₃ steht,
R¹⁵ für Wasserstoff, Fluor, Chlor oder OCH₃ steht,
mit den Maßgaben, dass R¹², R¹³, R¹⁴ und R¹⁵ nicht gleichzeitig Wasserstoff sind und dass, wenn R¹² Methyl ist, R¹³, R¹⁴ und R¹⁵ nicht gleichzeitig Wasserstoff sind,
(c) Pyridinderivaten mit den Formeln VII und VIII 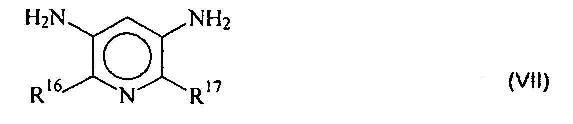 worin R¹⁶ und R¹⁷ gleich oder verschieden sein können und für Fluor, Chlor oder OCH₃ stehen, 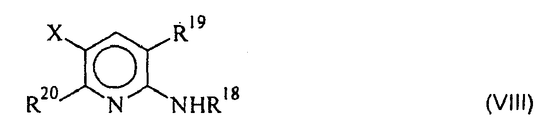 worin R¹⁸ für Wasserstoff, C₁-C₄-Alkyl oder C₁-C₄-Hydroxyalkyl steht,
R¹⁹ für OH oder NH₂ steht,
R²⁰ für Wasserstoff, C₁-C₄-Alkoxy oder NH₂ steht,
X für Wasserstoff oder OCH₃ steht,
mit den Maßgaben, dass, wenn R¹⁹ NH₂ ist, R¹⁸ und R²⁰ nicht gleichzeitig C₁-C₄₋Alkyl beziehungsweise Methoxy sind, und wenn R¹⁸ Wasserstoff ist, R¹⁹ und R²⁰ nicht gleichzeitig OH beziehungsweise Wasserstoff sind,
(d) Resorcinderivaten mit der Formel IX 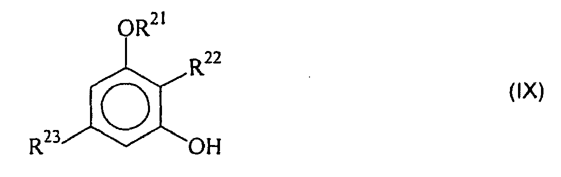 worin R²¹, R²² und R²³ gleich oder verschieden sein können und für Wasserstoff, C₁-C₄-Alkyl oder C₁-C₄-Hydroxyalkyl stehen,
mit den Maßgaben, dass R²¹, R²² und R²³ nicht gleichzeitig Wasserstoff sind, wenn R²¹ und R²³ Wasserstoff sind, R²² nicht Methyl ist, und wenn R²¹ für Methyl steht, R²² und R²³ nicht gleichzeitig Wasserstoff sind,
(e) Methylendioxybenzolderivaten, ausgewählt aus 3,4-Methylendioxyphenol, 3,4-Methylendioxyanilin und N-(2-Hydroxyethyl)-3,4-methylendioxyamlin,
(f) 3,4-Diaminobenzoesäure, und
C) aktivierten Carbonylverbindungen sowie weiteren als Entwickler oder Kuppler bekannte Substanzen.
zum Färben von keratinhaltigen Fasern.

12. Verfahren zum Färben von keratinhaltigen Fasern, insbesondere menschlichen Haaren, worin ein Färbemittel, enthaltend
A) mindestens ein Pyrimidinderivat mit der allgemeinen Formel I 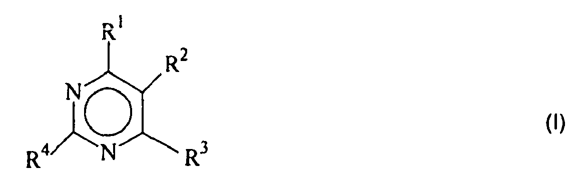 worin R¹, R², R³ und R⁴ gleich oder verschieden sein können und Wasserstoff, OH, NH₂ oder eine Gruppe NR⁵R⁶ sind, in der R⁵ und R⁶ gleich oder verschieden sein können und für C₁-C₄-Alkyl, C₁-C₄-Hydroxyalkyl mit primärer und/oder sekundärer Hydroxygruppe stehen,
wobei zwei der Reste R¹, R², R³ oder R⁴ zusammen einen gegebenenfalls substituierten 5- und 6-gliedrigen Heterocyclus mit einem oder zwei Stickstoff- und/oder Sauerstoffatom(en) im Molekül bilden können, mit der Maßgabe, dass mindestens zwei der Reste R¹, R², R³ oder R⁴ eine Gruppe NH₂ und/oder NR⁵R⁶ sind,
B) mindestens eine Verbindung ausgewählt aus der Gruppe bestehend
(a) m-Phenylenderivaten mit den Formeln II und III 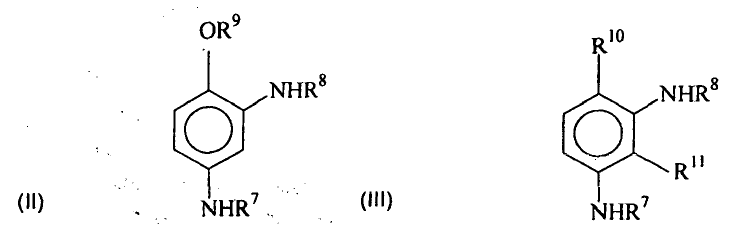 worin
R⁷ und R⁸ gleich oder verschieden sein können und für Wasserstoff, C₁₋C₄-Alkyl oder C₁-C₄-Hydroxyalkyl stehen,
R⁹ für C₁-C₄-Hydroxyalkyl oder einen Rest mit der allgemeinen Formel IV steht 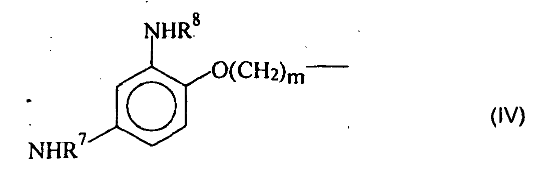 worin
R⁷ und R⁸ wie oben definiert sind und m eine ganze Zahl von 1 bis 4 bedeutet,
R¹⁰ für Wasserstoff oder einen Rest mit der allgemeinen Formel V steht 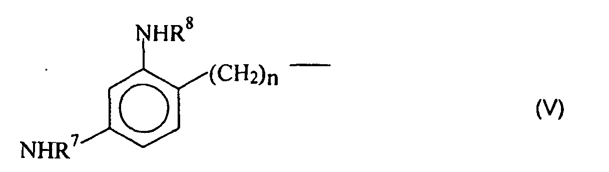 worin
R⁷ und R⁸ wie oben definiert sind und n eine ganze Zahl von 1 bis 4 bedeutet,
R¹¹ für Wasserstoff, C₁-C₄-Afkyl oder C₁-C₄-Hydroxyalkyl steht,
(b) m-Aminophenolderivaten 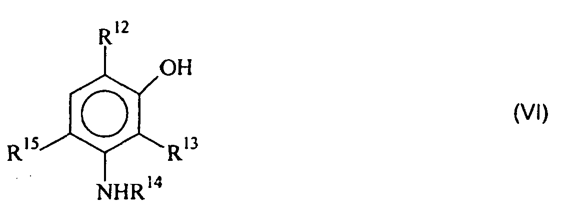 worin
R¹² für Wasserstoff oder C₁-C₄-Alkyl steht,
R¹³ für Wasserstoff, Fluor, Chlor, OCH₃ oder C₁-C₄-Alkyl steht,
R¹⁴ für Wasserstoff, C₁-C₄-Alkyl, C₁-C₄-Hydroxyalkyl oder OCF₃ steht,
R¹⁵ für Wasserstoff, Fluor, Chlor oder OCH₃ steht,
mit den Maßgaben, dass R¹², R¹³, R¹⁴ und R¹⁵ nicht gleichzeitig Wasserstoff sind und dass, wenn R¹² Methyl ist, R¹³, R¹⁴ und R¹⁵ nicht gleichzeitig Wasserstoff sind,
(c) Pyridinderivaten mit den Formeln VII und VIII 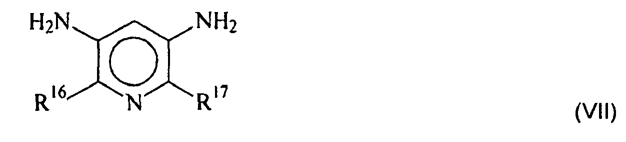 worin
R¹⁶ und R¹⁷ gleich oder verschieden sein können und für Fluor, Chlor oder OCH₃ stehen, 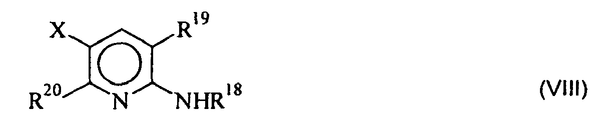 worin R¹⁸ für Wasserstoff, C₁-C₄-Alkyl oder C₁-C₄-Hydroxyalkyl steht,
R¹⁹ für OH oder NH₂ steht,
R²⁰ für Wasserstoff, C₁-C₄-Alkoxy oder NH₂ steht,
X für Wasserstoff oder OCH₃ steht,
mit den Maßgaben, dass, wenn R¹⁹ NH₂ ist, R¹⁸ und R²⁰ nicht gleichzeitig C₁-C₄-Alkyl beziehungsweise Methoxy sind, und wenn R¹⁸ Wasserstoff ist, R¹⁹ und R²⁰ nicht gleichzeitig OH beziehungsweise Wasserstoff sind,
(d) Resorcinderivaten mit der Formel IX 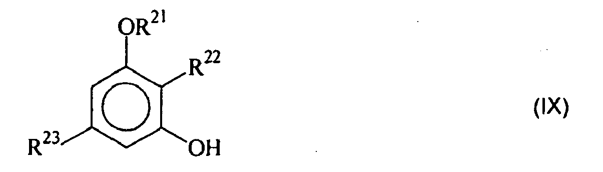 worin
R²¹, R²² und R²³ gleich oder verschieden sein können und für Wasserstoff, C₁-C₄-Alkyl oder C₁-C₄-Hydroxyalkyl stehen,
mit den Maßgaben, dass R²¹, R²² und R²³ nicht gleichzeitig Wasserstoff sind, wenn R²¹ und R²³ Wasserstoff sind, R²² nicht Methyl ist, und wenn R²¹ für Methyl steht, R²² und R²³ nicht gleichzeitig Wasserstoff sind,
(e) Methylendioxybenzolderivaten, ausgewählt aus 3,4-Methylendioxyphenol, 3,4-Methylendioxyanilin und N-(2-Hydroxyethyl)-3,4-methylendioxyanilin,
(f) 3,4-Diaminobenzoesäure, und
C) aktivierte Carbonylverbindungen sowie weitere als Entwickler oder Kuppler bekannte Substanzen
sowie übliche kosmetische Inhaltsstoffe auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. An agent for dyeing keratin fibers, comprising
A) at least one pyrimidine derivative of the general formula I 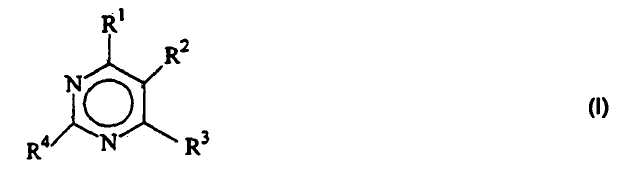 in which R¹, R², R³ and R⁴ may be identical or different and are hydrogen, OH, NH₂ or a group NR⁵R⁶, in which R⁵ and R⁶ may be identical or different and are C₁-C₄-alkyl, C₁-C₄-hydroxyalkyl having a primary and/or secondary hydroxyl group,
where two of the radicals R¹, R², R³ or R⁴ together can form an optionally substituted 5- and 6-membered heterocycle containing one or two nitrogen and/or oxygen atom(s) in the molecule,
with the proviso that at least two of the radicals R¹, R², R³ or R⁴ are a group NH₂ and/or NR⁵R⁶,
B) at least one compound chosen from the group consisting of
(a) m-phenylene derivatives of the formulae II and III 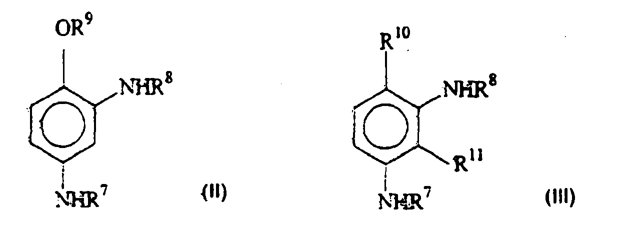 in which R⁷ and R⁸ may be identical or different and are hydrogen, C₁-C₄₋alkyl or C₁-C₄-hydroxyalkyl,
R⁹ is C₁-C₄-hydroxyalkyl or a radical of the general formula IV 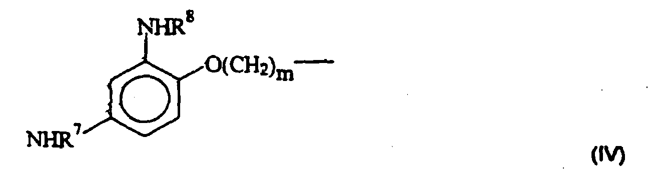 in which R⁷ and R⁶ are as defined above and m is an integer from 1 to 4,
R¹⁰ is hydrogen or a radical of the general formula V 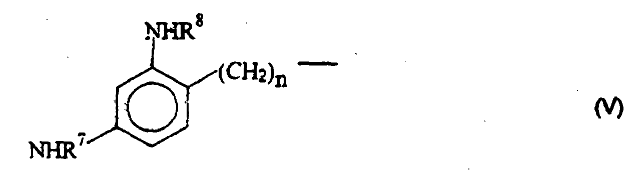 in which R⁷ and R⁸ are as defined above and n is an integer from 1 to 4,
R¹¹ is hydrogen, C₁-C₄-alkyl or C₁-C₄₋hydroxyalkyl,
(b) m-aminophenol derivatives 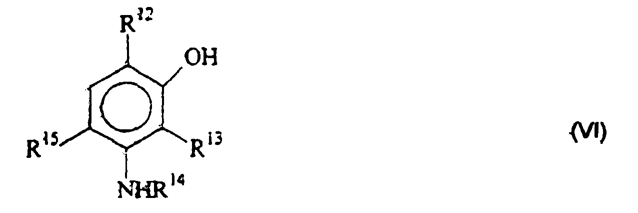 in which R¹² is hydrogen or C₁-C₄-alkyl,
R¹³ is hydrogen, fluorine, chlorine, OCH₃ or C₁-C₄-alkyl,
R¹⁴ is hydrogen, C₁-C₄-alkyl, C₁-C₄₋hydroxyalkyl or OCF₃,
R¹⁵ is hydrogen, fluorine, chlorine or OCH₃,
with the provisos that R¹², R¹³, R¹⁴ and R¹⁵ are not hydrogen at the same time and that, if R¹² is methyl, R¹³, R¹⁴ and R¹⁵ are not hydrogen at the same time,
(c) pyridine derivatives of the formulae VII and VIII 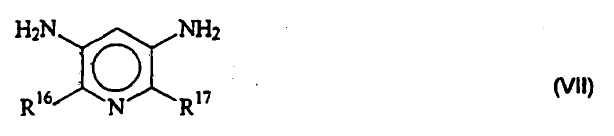 in which R¹⁶ and R¹⁷ may be identical or different and are fluorine, chlorine or -OCH₃, 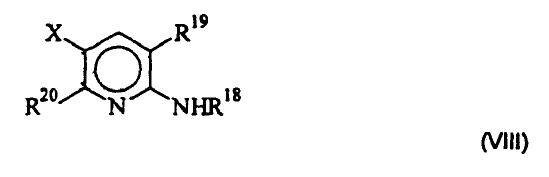 in which R¹⁶ is hydrogen, C₁-C₄-alkyl or C₁-C₄₋hydroxyalkyl,
R¹⁹ is OH or NH₂,
R²⁰ is hydrogen, C₁-C₄-alkoxy or NH₂,
X is hydrogen or OCH₃,
with the provisos that, if R¹⁹ is NH₂, R¹⁸ and R²⁰ are not C₁-C₄-alkyl or methoxy respectively at the same time, and if R¹⁸ is hydrogen, R¹⁹ and R²⁰ are not OH or hydrogen respectively at the same time,
(d) resorcinol derivatives of the formula IX 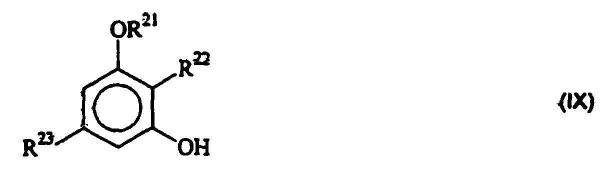 in which R²¹, R²² and R²³ may be identical or different and are hydrogen, C₁-C₄₋alkyl or C₁-C₄-hydroxyalkyl,
with the provisos that R²¹, R²² and R²³ are not hydrogen at the same time, if R²¹ and R²³ are hydrogen, R²² is not methyl, and if R²¹ is methyl, R²² and R²³ are not hydrogen at the same time,
(e) methylenedioxybenzene derivatives selected from 3,4-methylenedioxyphenol, 3,4-methylenedioxyaniline and N-(2-hydroxyethyl)-3,4-methylenedioxyaniline
(f) 3,4-diaminobenxoic acid and
C) activated carbonyl compounds and further substances known as developers or couplers.

2. The agent as claimed in claim 1, **characterized in that** 4-hydroxy-2,5,6-triaminopyrimidine, 2-hydroxy-2,5,6-triaminopyrimidine, 2,4,5,6-tetraaminopyrimidine, 5,6-diamino-2,4-dihydroxypyrimidine, 2,4-diamino-5,6-dihydroxypyrimidine, 4-dimethylamino-2,5,6-tetraminopyrimidine and any mixtures thereof are present as pyrimidine derivatives, of the formula I.

3. The agent as claimed in claim 1 or 2, **characterized in that** the pyrimidine derivatives of the formula I are present in an amount of from 0.03 to 65 mmol, in particular from 1 to 40 mmol, based on 100 g of the total dyeing agent.

4. The agent as claimed in any of claims 1 to 3, **characterized in that** the compounds of component B are chosen from the group 1,3-bis(2,4-diamino-phenoxypropane), 1,3-bis(2,4-diaminophenyl-propane), 2,4-diaminophenoxyethanol, 2,6-bis(2'-hydroxyethylamino) toluene, 3-amino-2-chloro-6-methylphenyl, 5-amino-4-chloro-2-methylphenol, 2,4-dichloro-3-aminophenol, 3,5-diamino-2,6-dimethoxypyridine, 5-methylresorcinol, 2,5-dimethylresorcinol, 3,4-methylenedioxyphenol, 3,4-methylenedioxyaniline, N-(2-hydroxyethyl)-3,4-methylenedioxyaniline and any mixtures thereof.

5. The agent as claimed in any of claims 1 to 4,
**characterized in that** the compounds of component B are present in an amount of, in each case, from 0.03 to 65 mmol, in particular from 1 to 40 mmol, in each case based on 100 g of the total dyeing agent.

6. The agent as claimed in any of claims 1 to 5, **characterized in that** the activated carbonyl compound is chosen from the group consisting of isatin, 5-chloroisatin, 5-bromoisatin, 6-bromoisatin, 5-nitroisatin, N-hydroxymethylisatin, N-allylisatin, 5-isatinsulfonic acid Na salt, glutaconaldehyde tetrabutylammonium salt, tribase aldehyde, malonaldehyde bis(dimethyl acetal), 4-hydroxy-3-methoxycinnanaldehyde, 1-piperidino-methylisatin, 1-diethylaminomethylisatin, glutaconaldehyde Na salt, 5-N-methylanilinopentadienyl, 2-chloro-3-hydroxymethylene-1-cyclohexene 1-aldehyde, N-(5-anilino-2,4-pentanedien-1-yl-idene)anilinium chloride, trans-β-(2-furyl)-acrolein, 2-nitro-1,3-indanedione, dehydroascorbic acid, 2-acetyl-1,3-cyclohexanedione, 7-dimethyl-amino-2,4,6-heptatrienylidene dimethylammonium perchlorate and 4-formyl-1-methylpyri-dinium benzenesulfonate.

7. The agent as claimed in any of claims 1 to 6, **characterized in that** one or more compounds chosen from 5,6-dihydroxyindole and its N-substituted C₁-C₄-alkyl and C₁-C₄-hydroxyalkyl derivatives, 5,6-dihydroxyindoline and its N-substituted C₁-C₄₋alkyl and C₁-C₄-hydroxyalkyl derivatives and the compounds known as developers, chosen from the group consisting of p-phenylenediamine, p-tolylenediamine, p-aminophenol, 4,4'-diaminodiphenylamine, 1,10-bis(2,5-diaminophenyl)-1,4,7,10-tetraoxydecane, 2,(2'-hydroxyethyl)-p-phenylenediamine, 2,6-dichloro-4-aminophenol, N,N-bis(2'-hydroxyethyl)-p-phenylenediamine, 3-methyl-4-aminophenol, 2-aminomethyl-4-aminophenol, 5-aminosalicylic acid, bis(2-hydroxy-5-aminophenyl)methane, 2-(2,5-diaminophenoxy)ethanol are also added.

8. The agent as claimed in any of claims 1 to 7, **characterized in that** it comprises anionic, zwitterionic or nonionic surfactants.

9. The agent as claimed in any of claims 1 to 8, **characterized in that** it is an air-oxidizable dyeing system.

10. The agent as claimed in any of claims 1 to 8, **characterized in that** [lacuna] comprises oxidizing agents chosen from the group H₂O₂, peroxydisulfate and percarbonate.

11. The use of a combination of
A) at least one pyrimidine derivative of the general formula I 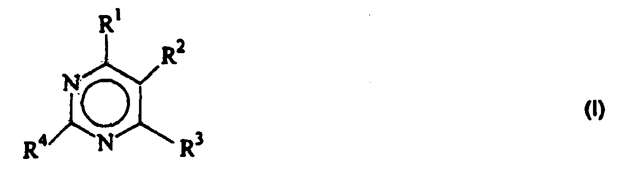 in which R¹, R², R³ and R⁴ may be identical or different and are hydrogen, OH, NH₂ or a group NR⁵R⁶, in which R⁵ and R⁶ may be identical or different and are C₁-C₄-alkyl, C₁-C₄-hydroxyalkyl having a primary and/or secondary hydroxyl group,
where two of the radicals R¹, R², R³ or R⁴ together can form an optionally substituted 5- and 6-membered heterocycle containing one or two nitrogen and/or oxygen atom(s) in the molecule,
with the proviso that at least two of the radicals R¹, R², R³ or R⁴ are a group NH₂ and/or NR⁵R⁶,
B) at least one compound chosen from the group consisting of
(a) m-phenylene derivatives of the formulae II and III 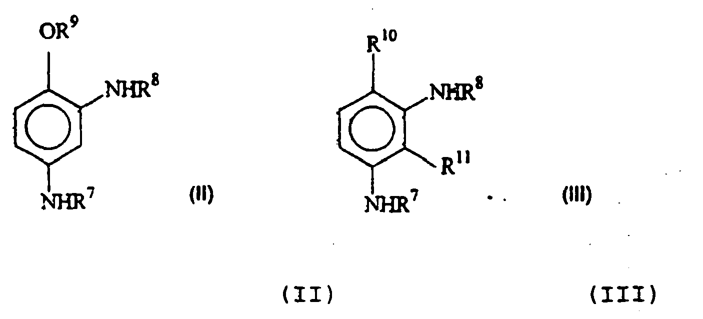 in which R⁷ and R⁸ may be identical or different and are hydrogen, C₁-C₄₋alkyl or C₁-C₄-hydroxyalkyl,
R⁹ is C₁-C₄-hydroxyalkyl or a radical of the general formula IV 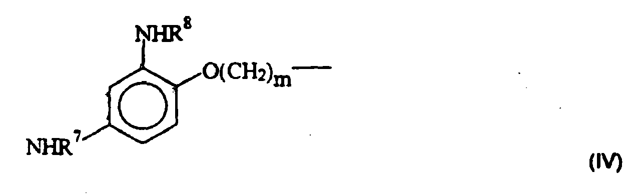 in which R⁷ and R⁸ are as defined above and m is an integer from 1 to 4,
R¹⁰ is hydrogen or a radical of the general formula V 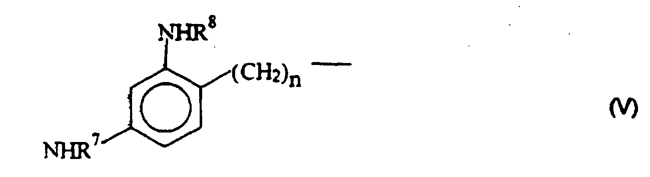 in which R⁷ and R⁸ are as defined above and n is an integer from 1 to 4,
R¹¹ is hydrogen, C₁-C₄-alkyl or C₁-C₄₋hydroxyalkyl,
(b) m-aminophenol derivatives 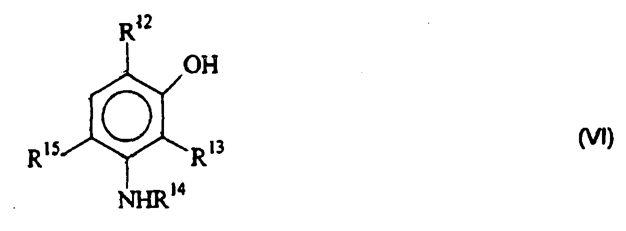 in which R¹² is hydrogen or C₁-C₄-alkyl,
R¹³ is hydrogen, fluorine, chlorine, OCH₃ or C₁-C₄-alkyl,
R¹⁴ is hydrogen, C₁-C₄-alkyl, C₁-C₄₋hydroxyalkyl or OCF₃, R¹⁵ is hydrogen, fluorine, chlorine or OCH₃,
with the provisos that R¹², R¹³, R¹⁴ and R¹⁵ are not hydrogen at the same time and that, if R¹² is methyl, R¹³, R¹⁴ and R¹⁵ are not hydrogen at the same time,
(c) pyridine derivatives of the formulae VII and VIII 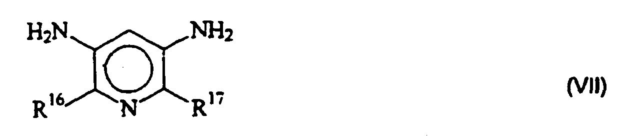 in which R¹⁶ and R¹⁷ may be identical or different and are fluorine, chlorine or OCH₃, 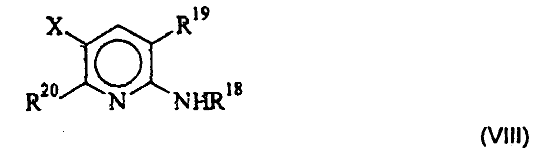 in which R¹⁸ is hydrogen, C₁-C₄-alkyl or C₁-C₄₋hydroxyalkyl,
R¹⁹ is OH or NH₂,
R²⁰ is hydrogen, C₁-C₄-alkoxy or NH₂,
X is hydrogen or OCH₃,
with the provisos that, if R¹⁹ is NH₂, R¹⁸ and R²⁰ are not C₁-C₄-alkyl or methoxy respectively at the same time, and if R¹⁸ is hydrogen, R¹⁹ and R²⁰ are not OH or hydrogen respectively at the same time,
(d) resorcinol derivatives of the formula IX 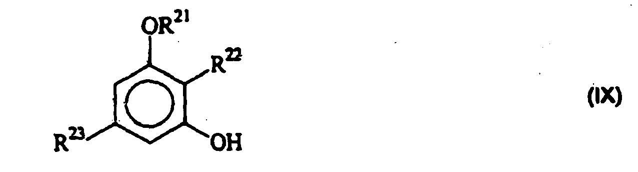 in which R²¹, R²² and R²³ may be identical or different and are hydrogen, C₁-C₄₋alkyl or C₁-C₄-hydroxyalkyl,
with the provisos that R²¹, R²² and R²³ are not hydrogen at the same time, if R²¹ and R²³ are hydrogen, R²² is not methyl, and if R²¹ is methyl, R²² and R²³ are not hydrogen at the same time,
(e) methylenedioxybenzene derivatives selected from 3,4-methylenedioxyphenol, 3,4-methylenedioxyaniline and N-(2-hydroxyethyl)-3,4-methylenedioxyaniline
(f) 3,4-diaminobenzoic acid, and
C) activated carbonyl compounds and further substances known as developers or couplers.
for dyeing keratin fibers.

12. A method of dyeing keratin fibers, in particular human hair, in which a dyeing agent comprising
A) at least one pyrimidine derivative of the general formula I 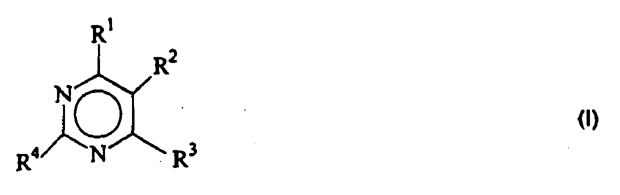 in which R¹, R², R³ and R⁴ may be identical or different and are hydrogen, OH, NH₂ or a group NR⁵R⁶, in which R⁵ and R⁶ may be identical or different and are C₁-C₄-alkyl, C₁-C₄-hydroxyalkyl having a primary and/or secondary hydroxyl group,
where two of the radicals R¹, R², R³ or R⁴ together can form an optionally substituted 5- and 6-membered heterocycle containing one or two nitrogen and/or oxygen atom(s) in the molecule,
with the proviso that at least two of the radicals R¹, R², R³ or R⁴ are a group NH₂ and/or NR⁵R⁶,
B) at least one compound chosen from the group consisting of
(a) m-phenylene derivatives of the formulae II and III 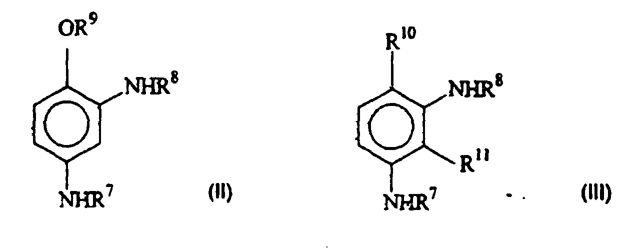 in which
R⁷ and R⁸ may be identical or different and are hydrogen, C₁-C₄-alkyl or C₁-C₄-hydroxyalkyl,
R⁹ is C₁-C₄-hydroxyalkyl or a radical of the general formula IV 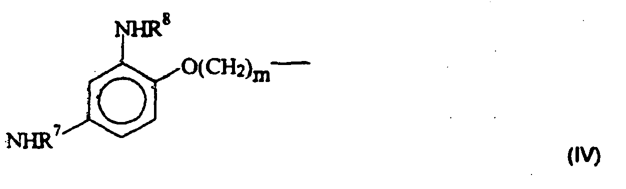 in which
R⁷ and R⁸ are as defined above and m is an integer from 1 to 4,
R¹⁰ is hydrogen or a radical of the general formula V 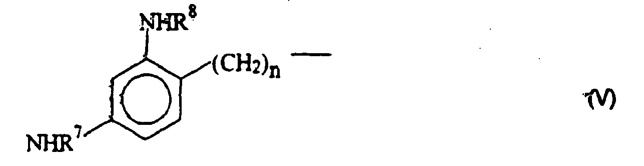 in which
R⁷ and R⁸ are as defined above and n is an integer from 1 to 4,
R¹¹ is hydrogen, C₁-C₄-alkyl or C₁-C₄₋hydroxyalkyl,
(b) m-aminophenol derivatives 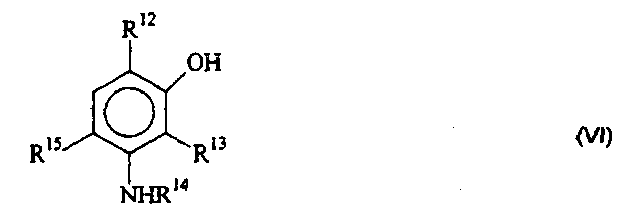 in which
R¹² is hydrogen or C₁-C₄-alkyl,
R¹³ is hydrogen, fluorine, chlorine, OCH₃ or C₁-C₄-alkyl,
R¹⁴ is hydrogen, C₁-C₄-alkyl, C₁-C₄-hydroxyalkyl or OCF₃,
R¹⁵ is hydrogen, fluorine, chlorine or OCH₃,
with the provisos that R¹², R¹³, R¹⁴ and R¹⁵ are not hydrogen at the same time and that, if R¹² is methyl, R¹³, R¹⁴ and R¹⁵ are not hydrogen at the same time,
(c) pyridine derivatives of the formulae VII and VIII 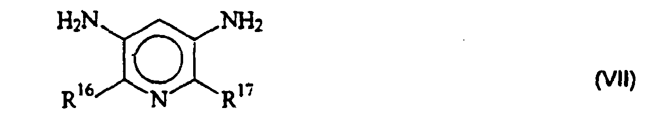 in which
R¹⁶ and R¹⁷ may be identical or different and are fluorine, chlorine or -OCH₃, 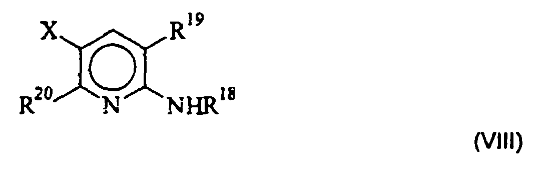 in which R¹⁸ is hydrogen, C₁-C₄-alkyl or C₁-C₄₋hydroxyalkyl,
R¹⁹ is OH or NH₂,
R²⁰ is hydrogen, C₁-C₄-alkoxy or NH₂,
X is hydrogen or OCH₃, with the provisos that, if R¹⁹ is NH₂, R¹⁸ and R²⁰ are not C₁-C₄-alkyl or methoxy respectively at the same time, and if R¹⁸ is hydrogen, R¹⁹ and R²⁰ are not OH or hydrogen respectively at the same time,
(d) resorcinol derivatives of the formula IX 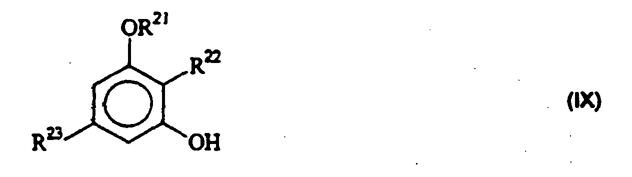 in which
R²¹, R²² and R²³ may be identical or different and are hydrogen, C₁-C₄-alkyl or C₁-C₄₋hydroxyalkyl,
with the provisos that R²¹, R²² and R²³ are not hydrogen at the same time, if R²¹ and R²³ are hydrogen, R²² is not methyl, and if R²¹ is methyl, R²² and R²³ are not hydrogen at the same time,
(e) methylenedioxybenzene derivatives selected from 3,4-methylenedioxyphenol, 3,4-methylenedioxyaniline and N-(2-hydroxyethyl)-3,4-methylenedioxyaniline
(f) 3,4-diaminobenzoic acid, and
C) activated carbonyl compounds and further substances known as developers or couplers.
and customary cosmetic ingredients, is applied to the keratin fibers, left on the fibers for a while, usually about 30 minutes, and then rinsed out again or washed out using a shampoo.

## Revendications

1. Agent de coloration de fibres kératiniques, comprenant
A) au moins un dérivé de pyrimidine de formule générale I 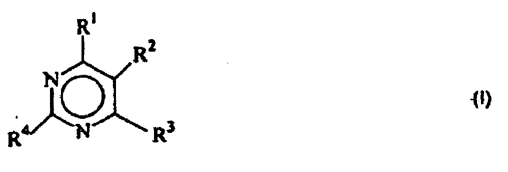 dans laquelle R¹, R², R³ et R⁴ peuvent être identiques ou différents et représentent un atome d'hydrogène, un groupe OH, NH₂, ou un groupe NR⁵R⁶, dans lequel R⁵ et R⁶ peuvent être identiques ou différents et représentent un groupe alkyle en C₁-C₄, hydroxy(alkyle en C₁-C₄) comprenant un groupe hydroxy primaire et/ou secondaire,
deux des groupes R¹, R², R³ ou R⁴ pouvant former ensemble un hétérocycle à 5 ou 6 maillons, éventuellement substitué, comprenant un ou deux atomes d'azote et/ou d'oxygène par molécule,
avec cette condition qu'au moins deux des groupes R¹, R², R³ ou R⁴ sont des groupes NH₂ et/ou NR⁵R⁶,
B) au moins un composé choisi dans le groupe constitué par
(a) des dérivés de m-phénylène de formules II et III 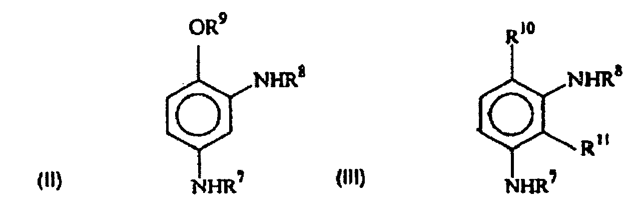 dans lesquelles R⁷ et R⁸ peuvent être identiques ou différents et représentent un atome d'hydrogène, un groupe alkyle en C₁-C₄ ou hydroxy(alkyle en C₁-C₄),
R⁹ représente un groupe hydroxy(alkyle en C₁-C₄) ou un groupe de formule générale IV 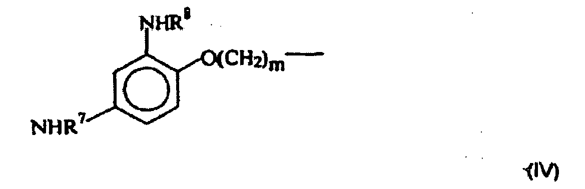 dans laquelle R⁷ et R⁸ sont définis comme ci-dessus et m représente un nombre entier de 1 à 4,
R¹⁰ représente un atome d'hydrogène ou un groupe de formule générale V 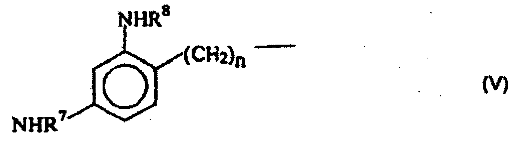 dans laquelle R⁷ et R⁸ sont définis comme ci-dessus et n représente un nombre entier de 1 à 4,
R¹¹ représente un atome d'hydrogène, un groupe alkyle en C₁-C₄ ou hydroxy(alkyle en C₁-C₄),
(b) des dérivés de m-aminophénol 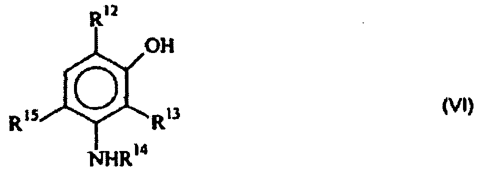 dans lesquels R¹² représente un atome d'hydrogène ou un groupe alkyle en C₁-C₄,
R¹³ représente un atome d'hydrogène, de fluor, de chlore, un groupe OCH₃ ou alkyle en C₁-C₄,
R¹⁴ représente un atome d'hydrogène, un groupe alkyle en C₁-C₄, hydroxy(alkyle en C₁-C₄) ou OCF₃,
R¹⁵ représente un atome d'hydrogène, de fluor, de chlore, ou un groupe OCH₃,
avec ces conditions que les groupes R¹², R¹³, R¹⁴ et R¹⁵ ne représentent pas en même temps un atome d'hydrogène et que, lorsque R¹² représente un groupe méthyle, R¹³, R¹⁴ et R¹⁵ ne représentent pas en même temps un atome d'hydrogène,
(c) des dérivés de pyridine de formules VII et VIII 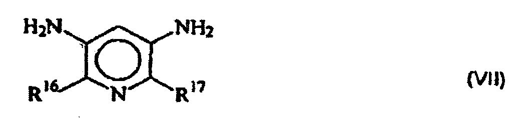 dans lesquelles R¹⁶ et R¹⁷ peuvent être identiques ou différents et peuvent représenter un atome de fluor, de chlore, ou un groupe -OCH₃, 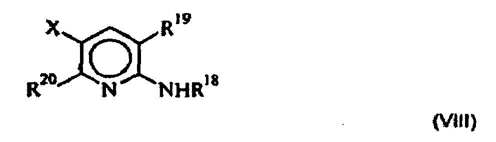 où R¹⁸ représente un atome d'hydrogène, un groupe alkyle en C₁-C₄ ou hydroxy(alkyle en C₁-C₄),
R¹⁹ représente un groupe OH ou NH₂,
R²⁰ représente un atome d'hydrogène, un groupe alcoxy en C₁-C₄ ou NH₂,
X représente un atome d'hydrogène ou un groupe OCH₃,
avec ces conditions que, lorsque R¹⁹ représente NH₂, R¹⁸ et R²⁰ ne représentent pas en même temps un groupe alkyle en C₁-C₄ ou méthoxy, et lorsque R¹⁸ représente un atome d'hydrogène, R¹⁹ et R²⁰ ne représentent pas en même temps un groupe OH ou un atome d'hydrogène,
(d) des dérivés de résorcinol de formule IX 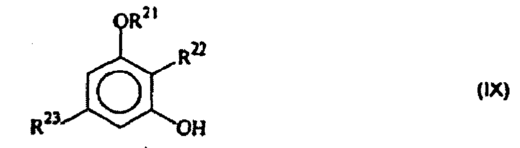 dans laquelle R²¹, R²² et R²³ peuvent être identiques ou différents et représentent un atome d'hydrogène, un groupe alkyle en C₁-C₄ ou hydroxy(alkyle en C₁-C₄),
avec ces conditions que les groupes R²¹, R²² et R²³ ne représentent pas en même temps un atome d'hydrogène, lorsque R²¹ et R²³ représentent un atome d'hydrogène, R²² ne représente pas un groupe méthyle, et
lorsque R²¹ représente un groupe méthyle, R²² et R²³ ne représentent pas en même temps un atome d'hydrogène,
(e) des dérivés de méthylènedioxybenzène, choisis parmi le 3,4-méthylène-dioxyphénol, la 3,4-méthylène-dioxyaniline et la N-(2-hydroxyéthyl)-3,4-méthylène-dioxyaniline,
(f) l'acide 3,4-diaminobenzoïque et
C) des composés carbonyle activés ainsi que d'autres substances connues comme développeurs ou coupleurs.

2. Agent selon la revendication 1, **caractérisé en ce que** comme dérivé de pyrimidine de formule I, il contient la 4-hydroxy-2,5,6-triaminopyrimidine, la 2-hydroxy-2,5,6-triaminopyrimidine, la 2,4,5,6-tétraaminopyrimidine, la 5,6-diamino-2,4-dihydroxypyrimidine, la 2,4-diamino-5,6-dihydroxypyrimidine, la 4-diméthyl-amino-2,5,6-triaminopyrimidine et les mélanges quelconques de ces composés.

3. Agent selon la revendication 1 ou 2, **caractérisé en ce qu'**il contient des dérivés de pyrimidine de formule I dans une proportion de 0,03 à 65 mmoles, en particulier de 1 à 40 mmoles pour 100 g de l'agent colorant total.

4. Agent selon l'une quelconque des revendications 1 à 3, **caractérisé en ce que** les composés du composant B sont choisis dans le groupe comprenant le 1,3-bis(2,4-diaminophénoxypropane), le 1,3-bis-(2,4-diaminophénylpropane), le 2,4-diaminophénoxyéthanol, le 2,6-bis-(2'-hydroxyéthylamino)-toluène, le 3-amino-2-chloro-6-méthylphénol, le 5-amino-4-chloro-2-méthylphénol, le 2,4-dichloro-3-aminophénol, la 3,5-diamino-2,6-diméthoxypyridine, le 5-méthyl-résorcinol, le 2,5-diméthylrésorcinol, le 3,4-méthylènedioxyphénol, la 3,4-méthylènedioxyaniline, la N-(2-hydroxyéthyl)-3,4-méthylènedioxyaniline et les mélanges quelconques de ces composés.

5. Agent selon l'une quelconque des revendications 1 à 4, **caractérisé en ce que** les composés du composant B sont contenus dans une proportion de 0,03 à 65, en particulier de 1 à 40 mmoles, pour 100 g de l'agent colorant total.

6. Agent selon l'une quelconque des revendications 1 à 5, **caractérisé en ce que** le composé carbonyle activé est choisi dans le groupe constitué par l'isatine, la 5-chloroisatine, la 5-bromoisatine, la 6-bromoisatine, la 5-nitroisatine, la N-hydroxyméthylisatine, la N-allylisatine, le sel de Na de l'acide 5-isatinesulfonique, le sel de tétrabutylammonium de l'aldéhyde glutaconique, l'aldéhyde tribasique, le bis-diméthylacétal de l'aldéhyde malonique, l'aldéhyde 4-hydroxy-3-méthoxy-cinnamique, la 1-pipéridino-méthylisatine, la 1-diéthylamino-méthylisatine, le sel de Na de l'aldéhyde glutaconique, le 5-N-méthylanilinopentadiénal, le 2-chloro-3-hydroxyméthylène-1-cyclohexène-1-aldéhyde, le chlorure de N-{5-anilino-2,4-pentanediène-1-ylidène)anilinium, la trans-β-(2-furyl)-acroleïne, la 2-nitro-1,3-indanedione, l'acide déhydroascorbique, la 2-acétyl-1,3-cyclohexanedione, le perchlorate de 7-diméthylamino-2,4,6-heptatriénylidène-diméthylammonium et le benzènesulfonate de 4-formyl-1-méthylpyridinium.

7. Agent selon l'une quelconque des revendications 1 à 6, **caractérisé en ce qu'**en outre, un ou plusieurs composés choisis parmi le 5,6-dihydroxyindol ainsi que ses dérivés N-substitués par des groupes alkyle en C₁-C₄ et hydroxy(alkyle en C₁-C₄), la 5,6-dihydroxyindoline ainsi que ses dérivés N-substitués par des groupes alkyle en C₁-C₄ et hydroxy(alkyle en C₁-C₄), et les composés connus en tant que développeurs, choisis dans le groupe constitué par la p-phénylènediamine, la p-toluylènediamine, le p-aminophénol, la 4,4'-diamino-diphénylamine, le 1,10-bis-(2,5-diaminophényl)-1,4,7,10-tétraoxydécane, la 2,(2'-hydroxyéthyl)-p-phénylène-diamine, le 2,6-dichloro-4-aminophénol, la N,N-bis-(2'-hydroxyéthyl)-p-phénylène-diamine, le 3-méthyl-4-aminophénol, le 2-aminométhyl-4-aminophénol, l'acide 5-amino-salicylique, le bis-(2-hydroxy-5-aminophényl)-méthane, le 2-(2,5-diamino-phénoxy)-éthanol y sont ajoutés.

8. Agent selon l'une quelconque des revendications 1 à 7, **caractérisé en ce qu'**il contient des tensioactifs anioniques, zwitterioniques ou non ioniques.

9. Agent selon l'une quelconque des revendications 1 à 8, **caractérisé en ce que** l'agent est constitué d'un système colorant oxydable à l'air.

10. Agent selon l'une quelconque des revendications 1 à 8, **caractérisé en ce que** l'agent oxydant est choisi dans le groupe comprenant H₂O₂, le peroxydisulfate et le percarbonate.

11. Utilisation d'une combinaison constituée par
A) au moins un dérivé de pyrimidine de formule générale I 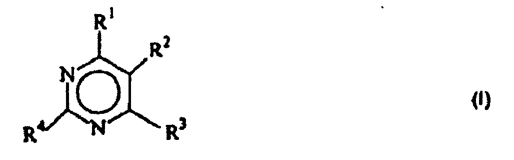 dans laquelle R¹, R², R³ et R⁴ peuvent être identiques ou différents et représentent un atome d'hydrogène, un groupe OH, NH₂, ou un groupe NR⁵R⁶, dans lequel R⁵ et R⁶ peuvent être identiques ou différents et représentent un groupe alkyle en C₁-C₄, hydroxy(alkyle en C₁-C₄) comprenant un groupe hydroxy primaire et/ou secondaire,
deux des groupes R¹, R², R³ ou R⁴ pouvant former ensemble un hétérocycle à 5 ou 6 maillons, éventuellement substitué, comprenant un ou deux atomes d'azote et/ou d'oxygène par molécule,
avec cette condition qu'au moins deux des groupes R¹, R², R³ ou R⁴ sont des groupes NH₂ et/ou NR⁵R⁶,
B) au moins un composé choisi dans le groupe constitué par
(a) des dérivés de m-phénylène de formules II et III 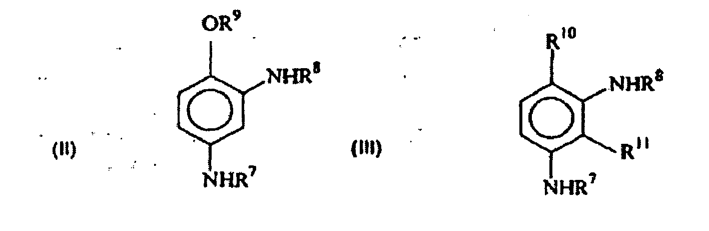 dans lesquelles R⁷ et R⁸ peuvent être identiques ou différents et représentent un atome d'hydrogène, un groupe alkyle en C₁-C₄ ou hydroxy(alkyle en C₁-C₄),
R⁹ représente un groupe hydroxy(alkyle en C₁-C₄) ou un groupe de formule générale IV 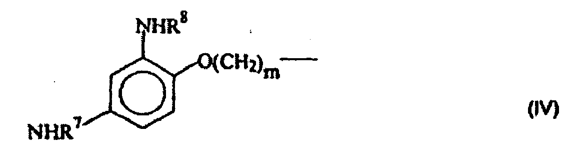 dans laquelle R⁷ et R⁸ sont définis comme ci-dessus et m représente un nombre entier de 1 à 4,
R¹⁰ représente un atome d'hydrogène ou un groupe de formule générale V 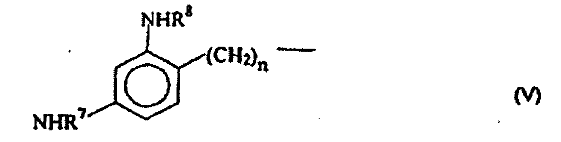 dans laquelle R⁷ et R⁸ sont définis comme ci-dessus et n représente un nombre entier de 1 à 4,
R¹¹ représente un atome d'hydrogène, un groupe alkyle en C₁-C₄ ou hydroxy(alkyle en C₁-C₄),
(b) des dérivés de m-aminophénol 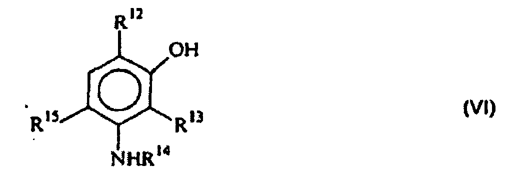 dans lesquels R¹² représente un atome d'hydrogène ou un groupe alkyle en C₁-C₄,
R¹³ représente un atome d'hydrogène, de fluor, de chlore, un groupe OCH₃ ou alkyle en C₁-C₄,
R¹⁴ représente un atome d'hydrogène, un groupe alkyle en C₁-C₄, hydroxy(alkyle en C₁-C₄) ou OCF₃,
R¹⁵ représente un atome d'hydrogène, de fluor, de chlore, ou un groupe OCH₃,
avec ces conditions que les groupes R¹², R¹³, R¹⁴ et R¹⁵ ne représentent pas en même temps un atome d'hydrogène et que, lorsque R¹² représente un groupe méthyle, R¹³, R¹⁴ et R¹⁵ ne représentent pas en même temps un atome d'hydrogène,
(c) des dérivés de pyridine de formules VII et VIII 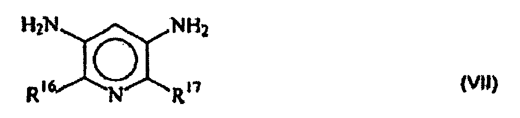 dans lesquelles R¹⁶ et R¹⁷ peuvent être identiques ou différents et peuvent représenter un atome de fluor, de chlore, ou un groupe -OCH₃, 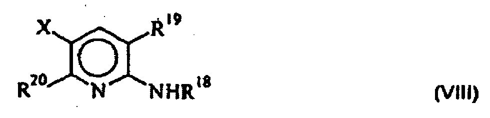 où R¹⁸ représente un atome d'hydrogène, un groupe alkyle en C₁-C₄ ou hydroxy(alkyle en C₁-C₄),
R¹⁹ représente un groupe OH ou NH₂,
R²⁰ représente un atome d'hydrogène, un groupe alcoxy en C₁-C₄ ou NH₂,
X représente un atome d'hydrogène ou un groupe OCH₃,
avec ces conditions que, lorsque R¹⁹ représente NH₂, R¹⁸ et R²⁰ ne représentent pas en même temps un groupe alkyle en C₁-C₄ ou méthoxy, et lorsque R¹⁸ représente un atome d'hydrogène, R¹⁹ et R²⁰ ne représentent pas en même temps un groupe OH ou un atome d'hydrogène,
(d) des dérivés de résorcinol de formule IX 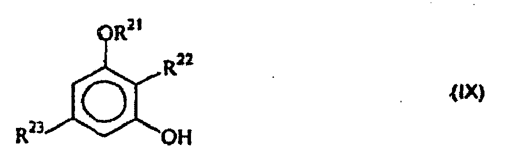 dans laquelle R²¹, R²² et R²³ peuvent être identiques ou différents et représentent un atome d'hydrogène, un groupe alkyle en C₁-C₄ ou hydroxy(alkyle en C₁-C₄),
avec ces conditions que les groupes R²¹, R²² et R²³ ne représentent pas en même temps un atome d'hydrogène, lorsque R²¹ et R²³ représentent un atome d'hydrogène, R²² ne représente pas un groupe méthyle, et lorsque R²¹ représente un groupe méthyle, R²² et R²³ ne représentent pas en même temps un atome d'hydrogène,
(e) des dérivés de méthylènedioxybenzène, choisis parmi le 3,4-méthylène-dioxyphénol, la 3,4-méthylène-dioxyaniline et la N-(2-hydroxyéthyl)-3,4-méthylène-dioxyaniline,
(f) l'acide 3,4-diaminobenzoïque et
C) des composés carbonyle activés ainsi que d'autres substances connues comme développeurs ou coupleurs
pour la coloration de fibres kératiniques.

12. Procédé de coloration de fibres kératiniques, en particulier de cheveux humains, dans lequel un colorant contenant
A) au moins un dérivé de pyrimidine de formule générale I 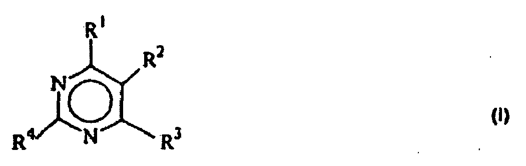 dans laquelle R¹, R², R³ et R⁴ peuvent être identiques ou différents et représentent un atome d'hydrogène, un groupe OH, NH₂, ou un groupe NR⁵R⁶, dans lequel R⁵ et R⁶ peuvent être identiques ou différents et représentent un groupe alkyle en C₁-C₄, hydroxy(alkyle en C₁-C₄) comprenant un groupe hydroxy primaire et/ou secondaire,
deux des groupes R¹, R², R³ ou R⁴ pouvant former ensemble un hétérocycle à 5 ou 6 maillons, éventuellement substitué, comprenant un ou deux atomes d'azote et/ou d'oxygène par molécule,
avec cette condition qu'au moins deux des groupes R¹, R², R³ ou R⁴ sont des groupes NH₂ et/ou NR⁵R⁶,
B) au moins un composé choisi dans le groupe constitué par
(a) des dérivés de m-phénylène de formules II et III 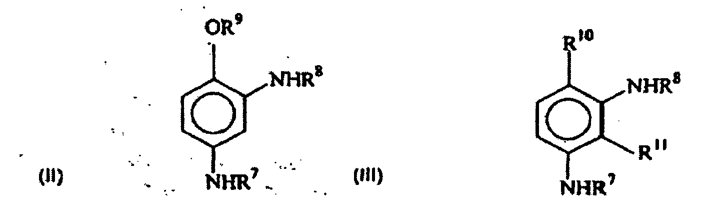 dans lesquelles
R⁷ et R⁸ peuvent être identiques ou différents et représentent un atome d'hydrogène, un groupe alkyle en C₁-C₄ ou hydroxy(alkyle en C₁-C₄),
R⁹ représente un groupe hydroxy(alkyle en C₁-C₄) ou un groupe de formule générale IV 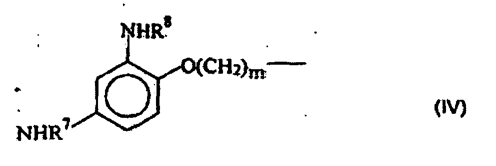 dans laquelle
R⁷ et R⁸ sont définis comme ci-dessus et m représente un nombre entier de 1 à 4,
R¹⁰ représente un atome d'hydrogène ou un groupe de formule générale V 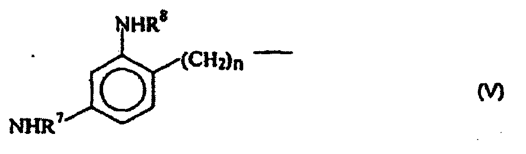 dans laquelle
R⁷ et R⁸ sont définis comme ci-dessus et n représente un nombre entier de 1 à 4,
R¹¹ représente un atome d'hydrogène, un groupe alkyle en C₁-C₄ ou hydroxy(alkyle en C₁-C₄),
(b) des dérivés de m-aminophénol 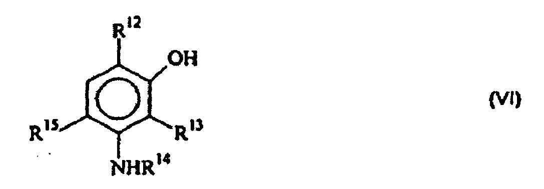 dans lesquels
R¹² représente un atome d'hydrogène ou un groupe alkyle en C₁-C₄,
R¹³ représente un atome d'hydrogène, de fluor, de chlore, un groupe OCH₃ ou alkyle en C₁-C₄,
R¹⁴ représente un atome d'hydrogène, un groupe alkyle en C₁-C₄, hydroxy(alkyle en C₁-C₄) ou OCF₃,
R¹⁵ représente un atome d'hydrogène, de fluor, de chlore, ou un groupe OCH₃,
avec ces conditions que les groupes R¹², R¹³, R¹⁴ et R¹⁵ ne représentent pas en même temps un atome d'hydrogène et que, lorsque R¹² représente un groupe méthyle, R¹³, R¹⁴ et R¹⁵ ne représentent pas en même temps un atome d'hydrogène,
(c) des dérivés de pyridine de formules VII et VIII 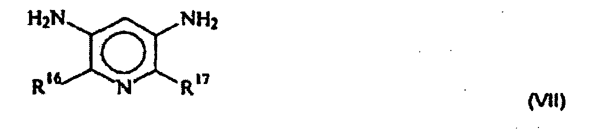 dans lesquelles
R¹⁶ et R¹⁷ peuvent être identiques ou différents et peuvent représenter un atome de fluor, de chlore, ou un groupe -OCH₃, 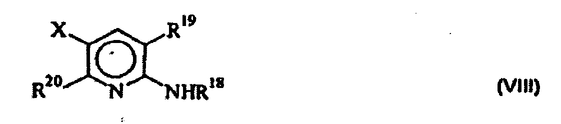 où R¹⁸ représente un atome d'hydrogène, un groupe alkyle en C₁-C₄ ou hydroxy(alkyle en C₁-C₄),
R¹⁹ représente un groupe OH ou NH₂,
R²⁰ représente un atome d'hydrogène, un groupe alcoxy en C₁-C₄ ou NH₂,
X représente un atome d'hydrogène ou un groupe OCH₃,
avec ces conditions que, lorsque R¹⁹ représente NH₂, R¹⁸ et R²⁰ ne représentent pas en même temps un groupe alkyle en C₁-C₄ ou méthoxy, et lorsque R¹⁸ représente un atome d'hydrogène, R¹⁹ et R²⁰ ne représentent pas en même temps un groupe OH ou un atome d'hydrogène,
(d) des dérivés de résorcinol de formule IX 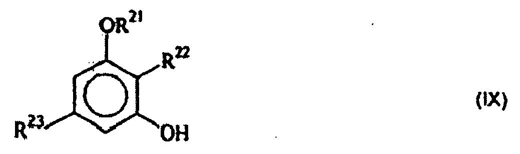 dans laquelle
R²¹, R²² et R²³ peuvent être identiques ou différents et représentent un atome d'hydrogène, un groupe alkyle en C₁-C₄ ou hydroxy(alkyle en C₁-C₄),
avec ces conditions que les groupes R²¹, R²² et R²³ ne représentent pas en même temps un atome d'hydrogène, lorsque R²¹ et R²³ représentent un atome d'hydrogène, R²² ne représente pas un groupe méthyle, et lorsque R²¹ représente un groupe méthyle, R²² et R²³ ne représentent pas en même temps un atome d'hydrogène,
(e) des dérivés de méthylènedioxybenzène, choisis parmi le 3,4-méthylène-dioxyphénol, la 3,4-méthylène-dioxyaniline et la N-(2-hydroxyéthyl)-3,4-méthylène-dioxyaniline,
(f) l'acide 3,4-diaminobenzoïque et
C) des composés carbonyle activés ainsi que d'autres substances connues comme développeurs ou coupleurs
ainsi que des constituants usuels des produits cosmétiques, est appliqué sur les fibres kératiniques, il y est laissé pendant un certain temps, généralement pendant environ 30 minutes, ensuite il en est éliminé par rinçage ou par lavage avec un shampooing.
